# FASCICULE DE BREVET EUROPEEN

(11) **EP 3 625 350 B1**
(45) Date de publication et mention de la délivrance du brevet: **23.12.2020**
(21) Numéro de dépôt: 18725212.7
(22) Date de dépôt: 16.05.2018
(51) Int. Cl.: C12P 3/00, C12P 5/02, C12M 1/107, C12M 1/00

(54) **DISPOSITIF ET PROCÉDÉ DE TRANSFORMATION DE MATIÈRES ORGANIQUES EN MÉLANGES DE MÉTHANE (CH4) ET/OU D'HYDROGÈN (H2) ET/OU DE DIXOYDE DE CARBONE (CO2), PAR COUPLAGE DE PROCÉDÉS THERMOCHIMIQUES ET BIOLOGIQUES**
VORRICHTUNG UND VERFAHREN ZUR UMWANDLUNG VON ORGANISCHEM MATERIAL IN MISCHUNGEN VON METHAN (CH4) UND / ODER HYDROGEN (H2) UND / ODER KOHLENDIOXID (CO2) DURCH KOPPLUNG VON THERMOCHEMISCHEN UND BIOLOGISCHEN PROZESSEN
DEVICE AND PROCESS FOR PROCESSING ORGANIC MATERIAL IN MIXED METHANE (CH4) AND / OR HYDROGEN (H2) AND / OR CARBON DIOXIDE (CO2) BY COUPLING THERMOCHEMICAL AND BIOLOGICAL PROCESSES

(30) Priorité: 16.05.2017 FR 1754307
(43) Date de publication de la demande: 25.03.2020
(73) Titulaire: YANNCO, 92800 Puteaux (FR); Institute National Polytechnique de Toulouse, 31029 Toulouse Cedex 4 (FR)
(72) Inventeur: MERCIER, Yann, 92800 Puteaux (FR); HEMATI, Mehrdji, 31860 Pins-Justaret (FR)
(74) Mandataire: Casalonga
(86) Numéro de dépôt international: PCT/EP2018/062797
(87) Numéro de publication internationale: WO 2018/210960

(56) Documents cités:
- WO-A1-2005/001976
- WO-A1-2015/003273
- US-A1- 2012 073 199
- GÖRLING MARTIN ET AL: "Bio-methane via fast pyrolysis of biomass", APPLIED ENERGY, vol. 112, 2013, pages 440-447, XP028731616, ISSN: 0306-2619, DOI: 10.1016/J.APENERGY.2013.01.002
- HÜBNER TOBIAS ET AL: "Integration of pyrolysis and anaerobic digestion - Use of aqueous liquor from digestate pyrolysis for biogas produc", BIORESOURCE TECHNOLOGY,, vol. 183, 14 février 2015 (2015-02-14), pages 86-92, XP029203391, ISSN: 0960-8524, DOI: 10.1016/J.BIORTECH.2015.02.037
- S Pérez ET AL: "SAFETY ASPECTS IN THE PRODUCTION AND SEPARATION OF HYDROGEN FROM BIOMASS", The 4th International Conference on Hydrogen Safety (ICHS) 2011 - Paper 129, 1 septembre 2011 (2011-09-01), pages 1-7, XP055441960, DOI: 10.1002/er.3381 Extrait de l'Internet: URL:http://conference.ing.unipi.it/ichs201 1/papers/129.pdf [extrait le 2018-01-18]
- S. T. CHAUDHARI ET AL: "Steam Gasification of Biomass-Derived Char for the Production of Carbon Monoxide-Rich Synthesis Gas", ENERGY & FUELS., vol. 15, no. 3, 1 mai 2001 (2001-05-01), pages 736-742, XP055498808, WASHINGTON, DC, US. ISSN: 0887-0624, DOI: 10.1021/ef000278c
- Robin Zwart et al.,: "Tar removal from low-temperature gasifiers", Report, ERA-NET Bioenergy project, 1 janvier 2010 (2010-01-01), XP055165370, Extrait de l'Internet: URL:http://www.ecn.nl/docs/library/report/ 2010/e10008.pdf [extrait le 2015-01-27]

## Description

La présente invention concerne un dispositif de transformation des matières organiques solides en méthane (CH₄), et/ou hydrogène (H₂) et/ou dioxyde de carbone (CO₂) comprenant en série une fonction de pyrogazéification, une fonction de reformage à la vapeur, une fonction de réaction de gaz à l'eau (appelée encore water gas shift en langue anglaise), une fonction de refroidissement et de lavage et une fonction de méthanation par voie biologique ou biométhanation.

L'invention concerne également un procédé de transformation des matières organiques solides en mélanges de méthane (CH₄) et/ou d'hydrogène (H₂) et/ou de dioxyde de carbone (CO₂) mettant en œuvre le procédé mentionné ci-avant.

La pyrolyse consiste généralement à traiter thermiquement des matières organiques en absence d'oxygène pour produire majoritairement un mélange de gaz incondensables, des huiles (ou goudrons) et du charbon (char). Les proportions entre ces trois phases principales dépendent avant tout des conditions opératoires, notamment de la température, de la vitesse de chauffage des matières et du temps de séjour dans le réacteur de pyrolyse. Par exemple, à une température moyenne (généralement autour de 300 à 500°C), et vitesse de chauffage rapide les teneurs en goudron sont favorisées, tandis que lorsque la vitesse de chauffage des matières organiques est lente alors les teneurs en char sont favorisées.

La gazéification consiste généralement à transformer en gaz les phases charbon et goudron produites lors de l'étape de pyrolyse, par ajout d'une quantité faible de matière oxydante, généralement de l'air, de l'oxygène pur ou encore de la vapeur d'eau. En effet, l'ajout de faibles quantités d'air ou d'oxygène pur provoque une oxydation partielle des huiles et des chars, ce qui a pour effet d'augmenter la température, parfois jusqu'à des valeurs supérieures à 1.200°C, voire 1.400°C, et permet de récupérer sous forme de gaz la plus grande partie possible de l'énergie contenue dans ces deux phases. En alternative ou en complément, l'ajout de vapeur d'eau est parfois réalisé pour accroitre le pouvoir oxydant de la réaction et favoriser la gazéification des huiles et/ou des chars, mais à la différence de la gazéification avec de l'air ou de l'oxygène pur, la gazéification avec de la vapeur d'eau n'est pas exothermique et ne génère donc pas de hausse de température.

Les réactions de pyrolyse et de gazéification peuvent avoir lieu au sein d'un même équipement, souvent simultanément, la gazéification transformant en gaz les huiles et les chars au fur et mesure qu'une réaction de pyrolyse est en train de se dérouler.

Le terme pyrogazéification recouvre les deux réactions de pyrolyse et de gazéification. En particulier, la frontière entre les termes « procédé de pyrolyse et procédé de gazéification » peut être fluctuante et dépend en partie des définitions préalables.

Au sens de la présente invention, le terme de pyrogazéification désigne donc indistinctement un procédé de pyrolyse et/ou de gazéification.

Les procédés de pyrogazéification génèrent des gaz de pyrogazéification (GP). Lorsque la pyrogazéification s'effectue à basse ou moyenne température (généralement entre 300°C et 900°C), le GP obtenu en sortie de l'unité de pyrogazéification, où il est généralement à une température comprise entre de 250 à 900°C, est généralement constitué de gaz incondensables, de gaz condensables de diverses natures, en particulier des huiles de pyrolyse vaporisées, ainsi que de différents types de particules fines solides, en particulier des particules de charbon. Ces différents produits sont présents dans le gaz de pyrogazéification en sortie du réacteur de pyrogazéification dans des proportions très variables selon les matières traitées et les conditions opératoires, en particulier en fonction de la température de la réaction.

Les matières susceptibles d'être valorisées dans les procédés de pyrogazéification sont des matières organiques de nature très diverse. Elles correspondent en général à des matières d'origine organique non fossiles, transformées ou non, ou bien à des matières organiques dérivées du pétrole.

Parmi les grandes familles de matières d'origine organique non fossiles opportunément valorisables par pyrogazéification, on peut citer principalement et de manière non exhaustive les biomasses végétales d'origine agricole (le bois, les pailles, les menu-pailles et autres résidus de culture, les cultures énergétiques etc.), certains déchets industriels banals non inertes (bois, cartons ...) et les déchets non fossiles issus d'une collecte sélective de matières organiques d'origine municipale, (déchets de bois, papiers, cartons, textiles, etc.) ou encore les déchets d'activités d'épuration (boues primaires de décantation ou de flottation de stations d'épuration, boues secondaires aérobies de stations d'épuration ou encore boues anaérobies de digestions des boues aérobies de stations d'épuration etc.).

Les matières dérivées du pétrole valorisables sont principalement des déchets d'objets en matière plastique (résidus urbains d'objets en plastique de la vie courante, sacs et autres emballages plastique, plastiques agricoles, pneus, résidus de décharges automobiles etc.).

En fonction du type de matières organiques à valoriser, de leur qualité, de leur quantité, de la qualité des gaz de pyrogazéification que l'on souhaite obtenir, de leur usage envisagé, et en fonction des technologies privilégiées, les types de procédés de pyrogazéification mis en œuvre sont nombreux et peuvent être très différents les uns des autres. De même, les modes de valorisation des gaz de pyrogazéification sont nombreux et variés.

Par exemple, les gaz de pyrogazéification peuvent être brûlés dans une chaudière pour produire de l'eau chaude ou de la vapeur, lesquelles peuvent être destinées à être mises en œuvre dans un procédé industriel, par exemple pour chauffer des utilités, ou encore dans une boucle de chaleur urbaine, pour chauffer des bâtiments.

En variante, les gaz de pyrogazéification peuvent aussi être brûlés dans une chaudière à vapeur pour alimenter en vapeur une turbine produisant de l'énergie électrique.

Par ailleurs, un gaz de pyrogazéification peut également être transformé en un mélange relativement pur de gaz incondensables, appelé gaz de synthèse, ou syngaz, constitué principalement de monoxyde de carbone (CO), de dihydrogène (H₂), communément appelé hydrogène, de méthane (CH₄) et de dioxyde de carbone (CO₂), afin d'alimenter un moteur de cogénération en vu de produire principalement de l'énergie électrique et de façon secondaire de la chaleur. Pour ce faire, le gaz de pyrogazéification doit généralement être préalablement refroidi et lavé pour obtenir du syngaz.

Cette dernière solution est intéressante car l'énergie électrique produite est plus facilement transportable et a généralement une plus grande valeur que l'énergie thermique. Ainsi, la production d'énergie sous forme d'énergie électrique facilement transportable permet d'envisager une décorrélation entre les lieux de production de l'énergie et les lieux où celle-ci est consommée.

Par « gaz de pyrogazéification », on entend donc au sens de la présente invention un mélange composé principalement de gaz incondensables et de gaz condensables de diverses natures, en particulier des huiles de pyrolyse vaporisées, obtenu suite à un procédé de pyrogazéification, l'ensemble entraînant et contenant éventuellement des particules fines solides, notamment des particules de charbon et/ou des cendres.

Par « gaz de synthèse » ou « syngaz », on entend au sens de la présente invention un mélange de gaz incondensables à température ambiante, constitué principalement d'hydrogène et/ou de méthane et/ou de dioxyde de carbone et/ou de monoxyde de carbone. Le syngaz peut aussi contenir des proportions, généralement faibles, d'autres gaz incondensables, comme de l'azote gazeux, ou des hydrocarbures plus lourds que le méthane, de type CnHm, ou encore du sulfure d'hydrogène (H₂S). Le syngaz est généralement obtenu après refroidissement et lavage du gaz de pyrogazéification. Lorsque le gaz de pyrogazéification est refroidi, la fraction condensable des gaz présents dans le gaz de pyrogazéification condense, entrainant souvent dans le liquide ainsi formé les particules fines solides présentes dans le GP. En plus d'une simple condensation des gaz condensables, le syngaz doit généralement être lavé ou épuré afin d'en augmenter la pureté. Pour ce faire, il existe des systèmes de lavage des gaz de pyrogazéification mis en œuvre par des procédés mécaniques, thermiques et/ou chimiques (filtres à manche, filtres à sable, filtres céramique, cyclones, lavage à l'eau, lavage à l'huile, lavage à la chaux, lavage électrostatique, etc.).

En d'autres termes, le gaz de synthèse ou syngaz correspond généralement à un gaz de pyrogazéification qui a été préalablement refroidi et lavé afin d'en minimiser la teneur en sous-produits solides, liquides ou condensables.

Par « huiles de pyrogazéification » (HP), on entend au sens de la présente invention l'ensemble des huiles (appelées aussi goudrons) et autres gaz condensés qui peuvent être séparés du syngaz et récupérés sous forme liquide ou pâteuse après refroidissement et lavage du gaz de pyrogazéification. Les huiles peuvent contenir des particules fines solides qui ont été entrainées dans les gaz de pyrogazéification lors de la réaction de pyrogazéification. Ainsi, une fois récupérées, les huiles de pyrogazéification sont généralement des produits liquides contenant une certaine quantité de matières solides en suspension. Souvent, les huiles de pyrogazéification condensées se séparent en 2 ou 3 fractions liquides et cireuses de masse volumique différentes.

Toutefois, la valorisation des gaz de pyrogazéification dans les chaudières ou les unités de cogénération peut poser des problèmes environnementaux, dans la mesure où ceux-ci peuvent contenir des matières dont la combustion peut produire des fumées toxiques, notamment en cas de pyrogazéification de déchets d'origine urbaine ou industrielle (cartons, plastiques, bois souillés, etc.). Dans ces conditions, des équipements de lavage des émissions gazeuses sont nécessaires afin de réduire leur caractère toxique, lesquels équipements sont généralement très coûteux en investissement et en exploitation.

De plus, les gaz de pyrogazéification peuvent être à l'origine de difficultés d'exploitation des équipements de valorisation, dans la mesure où les syngaz produits peuvent être de composition très variable selon les matières organiques traitées et le mode opératoire mis en œuvre. En particulier, les proportions de monoxyde de carbone, d'hydrogène, de méthane et de dioxyde de carbone sont souvent difficiles à contrôler et peuvent varier sensiblement. Dans ces conditions, le réglage et la constance de la combustion des syngaz dans les chaudières, et surtout dans les unités de cogénération, s'avèrent souvent problématiques.

Une autre difficulté majeure dans la valorisation des gaz de pyrogazéification, en particulier pour une valorisation en cogénération, résulte du caractère encrassant et corrosif des huiles de pyro gazéification.

En effet, les caractéristiques particulières des huiles contenues dans les gaz de pyrogazéification, en particulier le fait qu'elles sont constituées d'un mélange de nombreux types d'huiles différentes, dont certaines peuvent avoir une viscosité élevée, être instables et/ou être insolubles dans l'eau, rend généralement difficiles les manipulations des gaz de pyrogazéification. Il en résulte que l'utilisation des gaz de pyrogazéification présente un certain nombre de difficultés.

Tout d'abord, les GP sont des gaz très encrassant, en raison de la présence des huiles qu'ils contiennent, qui ont souvent une viscosité élevée, lesquels peuvent provoquer :
- l'encrassement des tuyauteries et des équipements avant l'introduction des gaz de pyrogazéification dans les chaudières,
- l'encrassement des brûleurs de chaudière, ce qui peut poser des difficultés de réglage de la combustion,
- l'encrassement des éventuels équipements de lavage de gaz, notamment avant l'introduction des GP dans des unités de cogénération, et
- l'encrassement des unités de cogénération.

Il résulte de ces difficultés que de nombreuses unités de pyrogazéification sont fréquemment arrêtées, certaines ayant été arrêtées de façon définitive.

De plus, les gaz de pyrogazéification présentent généralement l'inconvénient d'être corrosifs, en particulier en raison de la présence fréquente de sulfure d'hydrogène (H₂S) ou d'acide chlorhydrique (HCl), pouvant conduire à la corrosion des équipements décrits précédemment. Les sous-produits des gaz de pyrogazéification ont généralement un pH acide, souvent compris entre 2 et 4.

Ainsi, pour être utilisés en tant que carburant dans des unités de cogénération, les gaz de pyrogazéification doivent nécessairement être lavés et épurés de façon poussée afin d'obtenir des syngaz exempts de matières encrassantes et/ou corrosives, incompatibles avec le fonctionnement d'un moteur à explosion.

A cet effet, il existe des systèmes de lavage des gaz de pyrogazéification qui mettent en œuvre des procédés mécaniques, thermiques et/ou chimiques (filtres à manche, filtres à sable, filtres céramique, cyclones, lavage à l'huile, lavage à la chaux, lavage électrostatique, etc.). Toutefois, de tels systèmes sont généralement coûteux en termes d'investissement et d'exploitation, et sont souvent d'une fiabilité limitée, tant le caractère encrassant des gaz de pyrogazéification est élevé. Ainsi l'exploitation de certaines unités existantes de pyrogazéification est fréquemment suspendue en raison du fonctionnement insatisfaisant et du coût des systèmes de lavage mécaniques, thermiques et/ou chimiques.

Pour cette raison, le recours à un système de refroidissement et/ou de lavage du gaz de pyrogazéification par injection de celui-ci dans des liquides de refroidissement et/ou de lavage, de préférence de l'eau (nommés collectivement eaux de lavage), et/ou par aspersion de celui-ci avec des eaux de lavage, s'avère être une solution souvent plus efficace, plus fiable et moins coûteuse en termes d'investissement et d'exploitation.

Toutefois, de tels systèmes de refroidissement et/ou de lavage en voie liquide posent aussi des problèmes car le mélange des HP avec les eaux de lavage transforme ces eaux de lavage en déchets liquides, lesquels sont généralement difficiles ou impossibles à valoriser de manière rentable, et souvent trop coûteux à épurer et/ou à éliminer.

Une solution pourrait être la méthanisation des eaux de lavage des gaz de pyrogazéification contenant les huiles de pyrogazéification.

La méthanisation est une réaction de transformation par voie biologique anaérobie de matières organiques biodégradables solides ou liquides en un biogaz essentiellement composé de méthane et de dioxyde de carbone.

Les procédés de méthanisation sont mis en œuvre par l'introduction des matières organiques biodégradables à traiter dans des unités de méthanisation, généralement constituées d'un ou plusieurs digesteurs anaérobies. Ces digesteurs contiennent des digestats, constitués principalement d'eau, de matière organique non digérée, et d'un très grand nombre de bactéries anaérobies, appartenant à un très grand nombre de familles différentes, susceptibles de réaliser ensemble la fermentation anaérobie des matières organiques à transformer en biogaz. Les matières organiques à traiter sont mises en contact intime avec les bactéries présentes dans le digestat, généralement par agitation ou percolation du digestat dans les matières organiques, dans des conditions généralement contrôlées (température, concentrations etc.), généralement pendant plusieurs semaines.

Lorsque les matières organiques traitées ne contiennent pas de substances indésirables non biodégradables et/ou toxiques, en particulier pour les végétaux, le digestat excédentaire, produit par les unités de méthanisation, est un produit humide riche en matières organiques partiellement stabilisées et en micro-nutriments. Aussi, le digestat est souvent susceptible d'être utilisé ultérieurement comme amendement organique sur des terres agricoles.

La méthanisation peut s'appliquer notamment à tous les types de biomasses biodégradables ordinaires, tels que des produits ou des déchets issus de l'agriculture, des industries agroalimentaires ou encore des déchets alimentaires urbains ou de la restauration. La méthanisation peut aussi s'appliquer, sous certaines conditions plus strictes, à des déchets plus complexes, issus par exemple des industries chimiques, pétrochimiques, pharmaceutiques ou cosmétiques. La méthanisation pourrait donc probablement s'appliquer, sous certaines conditions, à certaines huiles de pyrogazéification, et en particulier aux eaux de lavage contenant les huiles de pyrogazéification.

Cependant, il s'avère que l'introduction d'eaux de lavage de gaz de pyrogazéification contenant une forte concentration d'huiles de pyrogazéification directement dans un réacteur de méthanisation pour les transformer par voie biologique en biogaz pose un certain nombre de problèmes. En effet, les huiles de pyrogazéification sont agressives pour les populations bactériennes, en raison du caractère toxique de certaines d'entre elles à certaines concentrations, et sont donc susceptibles d'inhiber les diverses réactions de transformation biologique du gaz de pyrogazéification en biogaz et/ou d'inhiber, voire d'éliminer, certaines familles de bactéries responsables de ces réactions.

Par exemple, les huiles présentes dans les gaz de la pyrolyse du bois, souvent appelées huiles de pyrolyse ou bio-huiles, ont souvent un pH de l'ordre de 2, très agressif pour les bactéries. Elles sont, en outre, constituées d'un mélange extrêmement complexe d'eau et de plus de deux cent composés oxygénés, parmi lesquels des fragments de lignine (lignine pyrolytique), des acides organiques (acétique, propionique, butyrique, formique, etc.), des aldéhydes et hydroxy-aldéhydes (formaldéhyde, acetaldéhyde, etc.), des cétones et hydroxy-cétones (acétone, cyclo-pentanone, etc.), des phénols (phénol, cresols, quaiacols, etc.), des alcools (méthanols, éthanol, etc.), des furanes (furfurals, etc.), etc. Or, il s'avère que la majorité de ces substances organiques sont toxiques pour certaines populations de bactéries au-dessus de certaines concentrations. De plus, les proportions de chacune de ces substances dans les bio-huiles varient sensiblement en fonction du type de bois traité, de la température de traitement, et du type de procédé mis en œuvre (avec ou sans injection d'air, de vapeur etc.).

Une solution à ces difficultés est proposée dans la demande brevet FR 1652182. La solution proposée est de laver les gaz de pyrogazéification et séparer le syngaz et les huiles par voie liquide, puis de traiter le liquide de lavage chargé d'huiles de pyrolyse dans une unité de méthanisation.

Pour résoudre le problème de la toxicité des huiles de pyrogazéification sur certaines populations de bactéries présentes dans les digestats liquides, la demande de brevet FR 1652182 propose de mettre en œuvre, à proximité de l'unité de méthanisation des huiles de pyrolyse, une autre unité de méthanisation ayant pour but de produire un digestat liquide de bonne qualité afin d'alimenter l'unité de méthanisation des huiles de pyrogazéification avec le digestat liquide. Dans ce contexte, la fonction première de l'unité de production de digestat liquide est d'alimenter l'unité de méthanisation des huiles de pyrogazéification en digestat liquide en quantité suffisante pour que la concentration de chacune des substances qui composent les huiles de pyrogazéification soit suffisamment faible pour rendre possible la méthanisation des huiles de pyrogazéification.

Plus précisément, l'alimentation en digestat liquide de l'unité de méthanisation des huiles de pyrogazéification permet de diluer la concentration de chacune des substances composant les huiles de pyrogazéification dans le digestat contenu dans l'unité de méthanisation, de façon suffisamment importante pour que chacune de ces substances se retrouve à une concentration assez faible pour rendre ladite substance non toxique pour les bactéries présentes dans ledit digestat ce qui permet de diminuer les risques d'inhibition de l'activité de ces bactéries.

En effet, la plupart des substances présentes dans les huiles de pyrogazéification, par exemple les diverses huiles présentes dans les bio-huiles de bois, sont toxiques au-dessus de certaines concentrations pour la majorité des familles de bactéries présentes dans le digestat agissant dans une unité de méthanisation. A l'inverse, la plupart des substances présentes dans les huiles de pyrogazéification sont non toxiques lorsqu'elles se trouvent en solution dans le digestat en dessous de certains seuils de concentration (appelé seuil de toxicité) et une grande partie de ces substances sont en outre biodégradables lorsqu'elles sont mises en présence de bactéries adaptées à la biodégradation de ces substances.

Toutefois la solution proposée dans la demande brevet FR 1652182 pourrait encore, dans certains cas, se révéler insuffisante.

En effet, certaines huiles de pyrogazéification peuvent contenir des matières organiques non biodégradables et impropres à un usage agricole, notamment en cas de pyrogazéification de déchets d'origine urbaine (plastiques, bois souillés, etc.). Dans ces conditions, la totalité des digestats produits par le dispositif est rendue inutilisable en usage agricole, ce qui génère des coûts prohibitifs d'épuration, voire d'élimination de ces digestats.

En outre, certaines huiles de pyrogazéification peuvent contenir une majorité de matières organiques non biodégradables ou faiblement dégradables, donnant lieu à un faible rendement de transformation de ces huiles de pyrogazéification en biogaz, et, par conséquent, à un faible rendement de valorisation énergétique des gaz de pyrogazéification.

Ainsi la solution consistant à transformer un gaz de pyrogazéification en un syngaz utilisable dans une unité de cogénération génère des contraintes importantes en matière de coûts d'investissement, de coûts d'exploitation et de fiabilité.

Une valorisation des gaz de pyrogazéification encore plus intéressante que la production de chaleur ou la cogénération, peut être la méthanation des syngaz. Il s'agit de laver les gaz de pyrogazéification pour séparer les huiles de pyrogazéification du syngaz, et de transformer les syngaz obtenus en biogaz par le biais de procédés de méthanation.

Par procédé de « méthanation » on nomme tout procédé visant à transformer de l'hydrogène en biogaz par réaction avec du monoxyde de carbone et/ou du dioxyde de carbone. Les réactions mises en œuvre sont les suivantes :

CO + 3H₂ → CH₄ + H₂O

CO₂ + 4H₂ → CH₄ + 2H₂O

Au sens de la présente invention, par méthanation, on désigne les procédés ayant pour objet la transformation tout type de gaz contenant de l'hydrogène et au moins du monoxyde de carbone et/ou du dioxyde de carbone. C'est le cas, en particulier du syngaz, qui est un mélange de gaz incondensables à température ambiante, constitué principalement de monoxyde de carbone et/ou d'hydrogène et/ou de méthane et/ou de dioxyde de carbone. L'hydrogène présent dans le syngaz produit du méthane par réaction avec du monoxyde de carbone et/ou du dioxyde de carbone.

Plus largement, en présence d'excès de dioxyde de carbone, au sens de la présente invention, par méthanation, on désigne les procédés ayant pour objet la transformation de syngaz en biogaz, lequel biogaz est un mélange essentiellement composé de méthane et de dioxyde de carbone. Le biogaz peut être lui-même transformé en biométhane suite à une opération d'épuration, ou purification (ou encore « upgrading » en langue anglaise), ou encore séparation de gaz.

L'épuration du biogaz est un procédé de séparation du méthane et du dioxyde de carbone (et des autres gaz minoritaires) qui permet la production d'un biométhane assez pur pour le rendre apte à être injecté dans les réseaux de gaz naturel (généralement > 97%), ou à servir de carburant dans les transports routiers (généralement > 85%).

Il existe deux groupes de procédés de méthanation, les procédés de méthanation par transformation catalytique et les procédés de méthanation par voie biologique, ou biométhanation.

Les procédés de méthanation par transformation catalytique sont largement décrits dans la littérature scientifique. Ils ont fait l'objet de nombreuses expériences de laboratoire, et ont été mis en œuvre concrètement par la construction de quelques unités de taille industrielle qui fonctionnent depuis peu de temps (par exemple l'unité Gobigas, à Gotteborg en Suède, ou encore l'unité expérimentale Gaya, à Lyon). Voir aussi le document de Görling et al., "Bio-methane via fast pyrolysis of biomass" Applied Energy, 112 (2013), 440-447.

Cependant, la méthanation par transformation catalytique reste une technologie très coûteuse en investissement et en exploitation car elle repose sur des procédés faisant appel à des températures et des pressions élevées, et qui nécessitent l'utilisation de catalyseurs coûteux, comme par exemple le nickel, qu'il faut, en outre, régénérer de façon régulière.

De plus, pour que la méthanation du syngaz par transformation catalytique fonctionne correctement, il est nécessaire de laver et d'épurer au préalable le gaz de pyrogazéification de façon extrêmement poussée, car les catalyseurs utilisés sont très sensibles aux impuretés. Une telle opération engendre une nouvelle fois des contraintes de coûts d'investissement et de coûts d'exploitation pouvant remettre en cause la viabilité économique de ces procédés.

Les procédés de méthanation par voie biologique, dits procédés de biométhanation, sont également décrits dans la littérature scientifique et ont été validés par quelques expériences en laboratoire et quelques pilotes expérimentaux.

De tels procédés consistent à introduire du gaz contenant de l'hydrogène et au moins du monoxyde de carbone et/ou du dioxyde de carbone, en particulier le syngaz, dans un réacteur de biométhanation afin de le transformer en biogaz par voie biologique anaérobie.

Par exemple, dans la demande de brevet WO2015003273, un digesteur anaérobie est alimenté en charge d'alimentation, par exemple en boue provenant d'une usine de traitement des eaux usées municipales, et produit un digestat. Le digestat est déshydraté jusqu'à l'obtention d'un gâteau. Le gâteau peut être séché un peu plus, par exemple dans un séchoir thermique. Le gâteau est traité dans un système de pyrolyse afin de produire un gaz de synthèse et un charbon de biomasse. Le gaz est envoyé au même digesteur ou à un digesteur différent afin d'augmenter sa production de méthane. Le charbon de biomasse peut être utilisé en tant que produit d'amélioration du sol.

La demande de brevet US2012/073199 divulgue un procédé de pyro-biométhanisation dont les boues provenant d'un digesteur anaérobie sont déshydratées et soumises à la pyrolyse. La sortie de pyrolyse est refroidie et condensée et séparée en gaz, liquides et solides. Le pyrogaz produit comprend du CO2 du CO, de l' H2 et du CH4. Les liquides condensés de la pyrolyse, les "acides pyroligneux", comprennent divers alcools, acides organiques, cétones et autres substances organiques. Les solides résiduels comprennent de cendres et du charbon. L'acide pyroligneux (facultativement avec les pyrogaz) est recyclé dans le digesteur anaérobie et soumis à une digestion anaérobie avec des microorganismes méthanogènes pour produire un gaz combustible riche en méthane.

Finalement, le document de Hübner et Mumme, 2015 ("Intégration of pyrolysis and naerobic digestion - Use of aqueous liquor from digestate pyrolysis for biogas production". Bioresource Technology, 183: 86-92) décrit un dispositif et un procédé de transformation par voie biologique de gaz de pyrogazéification en biogaz comprenant: (a) un bioréacteur anaérobie pour la production d'un digestat anaérobie et un système de séparation de phase pour produire un digestat solide pour son utilisation dans un procédé de pyrolyse et une phase liquide (contenant des bactéries anaérobies) maintenu a températures mésophiles pendant 2 semaines, et (b) une unité fonctionnelle comprenant un pyrolyseur avec (i) une fonction de refroidissement du gaz de pyrogazéification et de lavage, (ii) une fonction de méthanisation d'au moins une partie des sous-produits du gaz de pyrogazéification afin de former substances non-condensables, du gaz et du goudron, et la liqueur de pyrolyse. Le bioréacteur anaérobie est alimentée par la phase aqueuse du liqueur de pyrolyse pour réaliser une biométhanation.

Par procédé de « biométhanation » on nomme tout procédé visant à transformer par voie biologique de l'hydrogène en biogaz par réaction avec du monoxyde de carbone et/ou du dioxyde de carbone conformément aux réactions décrites précédemment.

Au sens de la présente invention, par biométhanation, on désigne les procédés ayant pour objet la transformation par voie biologique de tout type de gaz contenant de l'hydrogène et au moins du monoxyde de carbone et/ou du dioxyde de carbone, en particulier du syngaz, en méthane.

Au cours de la réaction de biométhanation les réactions chimiques principales sont :

(1) 4CO + 2H₂O→ CH₄ + 3CO₂

(2) CO₂ + 4H₂ → CH₄ + 2H₂O

(3) 2CO + 2H₂ → CH₄ + CO₂

Un des avantages principaux de la biométhanation par rapport à la méthanation catalytique est que la biométhanation tolère la présence d'une plus grande quantité d'impuretés dans les gaz mentionnés ci-avant, notamment le syngaz. Il en résulte que les équipements de lavage des gaz de pyrogazéification placés en amont d'une unité de biométhanation nécessitent moins d'efficacité que ceux qui sont placés en amont d'une unité de méthanation catalytique.

Malgré cette plus grande tolérance aux impuretés des unités de biométhanation, il n'est probablement pas envisageable d'introduire des gaz de pyrogazéification non lavés directement dans une unité de biométhanation (sauf éventuellement si le volume de gaz de pyrogazéification non lavés introduit est très faible par rapport au volume de l'unité de biométhanation).

En effet, le gaz de pyrogazéification non lavé est très agressif pour les populations bactériennes, en raison de la température élevée du gaz en sortie de l'unité de pyrogazéification et surtout de la présence des huiles de pyrogazéification dans celui-ci.

Comme indiqué précédemment, les huiles de pyrogazéification peuvent être toxiques à certaines concentrations, et donc susceptibles d'inhiber les diverses réactions de transformation biologique du gaz de pyrogazéification en biogaz et de tuer certaines familles de bactéries responsables de ces réactions.

De plus, le gaz de pyrogazéification peut contenir des matières organiques non biodégradables impropres à un usage agricole, notamment en cas de pyrogazéification de déchets d'origine urbaine (plastiques, bois souillés, etc.). Dans ces conditions, la totalité du ou des digestats, produite par le dispositif objet de l'invention, serait rendue inutilisable en usage agricole, ce qui générerait des coûts prohibitifs d'épuration, voire d'élimination de ces digestats.

Pour disposer d'un gaz, notamment du syngaz, propre à être introduit dans l'unité de biométhanation, il est, ici encore, nécessaire de procéder à un refroidissement et un lavage préalable du gaz de pyrogazéification afin de séparer les sous-produits du gaz de pyrogazéification, dont les huiles de pyrogazéification, et d'obtenir un gaz, en particulier un syngaz, moins agressif pour la flore bactérienne de l'unité de biométhanation, ce qui engendre, une nouvelle fois, des contraintes en termes de coûts d'investissement et de coûts d'exploitation, et peut limiter la fiabilité de la chaine de ces procédés.

Cependant, le gaz, même convenablement lavé et épuré, peut encore être agressif pour certaines familles de bactéries au-dessus de certaines concentrations, car le monoxyde de carbone contenu dans le gaz, notamment le syngaz, est toxique pour certaines familles de bactéries au dessus de certaines concentrations de dilution dans les digestats. Ainsi la fiabilité des procédés de biométhanation reste faible et comporte des risques élevés d'inhibition ou de mortalité pour certaines populations bactériennes, notamment les plus fragiles.

Certaines publications suggèrent de réaliser la transformation biologique du gaz de pyrogazéification en biogaz à partir d'une unité de méthanisation ordinaire, alimentée en biomasse, dont les digestats, produits par l'unité de méthanisation, seraient séchés et envoyés dans une unité de pyrogazéification afin d'y être gazéifiés. Le gaz de pyrogazéification ainsi produit serait donc envoyé dans l'unité de méthanisation pour être transformé en biogaz. Ainsi, dans cette solution, l'unité de méthanisation de biomasse jouerait également le rôle d'unité de transformation biologique du gaz de pyrogazéification, dans laquelle celui-ci serait transformé en biogaz. En d'autres termes, cette unité serait le lieu à la fois d'une réaction de méthanisation de la biomasse et de transformation biologique du gaz de pyrogazéification en biogaz. Cette transformation biologique du gaz de pyrogazéification en biogaz peut être décomposée en une réaction de méthanisation des huiles de pyrogazéification condensées contenues dans le gaz de pyrogazéification et une réaction de biométhanation du syngaz.

Un tel procédé réalisant à la fois la méthanisation de biomasses ordinaires et la transformation biologique du gaz de pyrogazéification brut, c'est-à-dire sans traitement préalable, serait théoriquement possible si, d'une part, le volume des gaz de pyrogazéification introduit dans l'unité de méthanisation était très faible par rapport au volume de l'unité de méthanisation, de telle sorte que les huiles de pyrogazéification présentes dans le gaz de pyrogazéification introduit soient suffisamment diluées dans le digestat de méthanisation pour ne pas être toxiques pour les bactéries présentes dans ce digestat, et si, d'autre part, la totalité des huiles de pyrogazéification était biodégradable.

Dans la réalité, il est très improbable que la totalité des huiles de pyrogazéification soit biodégradable, et un tel procédé réalisant à la fois la méthanisation de biomasses ordinaires et la transformation biologique du gaz de pyrogazéification brut, c'est-à-dire sans traitement préalable en biogaz semble difficile à mettre en œuvre dans un usage de dimension industrielle. En effet, les matières non biodégradables, dont les éléments minéraux, solides ou liquides, qui seraient entraînées dans le gaz de pyrogazéification, se retrouveraient dans l'unité de méthanisation, puis seraient entraînées dans les digestats produits par l'unité de méthanisation, lesquels seraient séchés, puis transférés dans l'unité de pyrogazéification, où ces matières non biodégradables présentes dans le gaz de pyrogazéification seraient de nouveau entraînées dans le gaz de pyrogazéification et transférées dans l'unité de méthanisation.

Ces matières non biodégradables seraient donc prisonnières d'une boucle de recirculation fermée, conduisant à une concentration croissante de celles-ci, notamment dans l'unité de méthanisation, aboutissant inévitablement à des concentrations inhibitrices pour la flore bactérienne, et finalement à une toxicité létale pour les bactéries.

En outre, dans cette configuration, l'augmentation de la concentration des matières éventuellement non biodégradables et indésirables dans l'unité de méthanisation, provenant du gaz de pyrogazéification, pourrait aussi rendre impropre à un usage agricole le digestat produit par l'unité de méthanisation. En effet, les autorisations d'épandage agricole dépendent de la concentration des matières indésirables dans les produits à épandre.

De plus, les bactéries anaérobies ont généralement besoin de s'acclimater aux produits qu'elles doivent digérer et se spécialiser pour réaliser convenablement cette digestion. En effet, certains produits à digérer peuvent être inhibiteurs ou toxiques à certaines concentrations pour certaines familles de bactéries, tandis qu'ils peuvent au contraire favoriser l'activité d'autres familles de bactéries. Les réactions biologiques qui interviennent dans la méthanisation de biomasses ordinaires, ou dans la transformation biologique du gaz de pyrogazéification en biogaz, ou encore dans la biométhanation du syngaz mettent en œuvre des processus biologiques différents, qui nécessitent une adaptation des bactéries, notamment par l'acclimatation et la sélection des familles de bactéries les plus adaptées pour la digestion de chaque type de substance à traiter. Or les substances à transformer dans les biomasses ordinaires, dans le gaz de pyrogazéification et dans le syngaz sont de nature très différente. Il existe une probabilité élevée qu'une opération simultanée, dans la même cuve, de méthanisation de biomasses ordinaires, de méthanisation des huiles de pyrogazéification et de biométhanation des syngaz, donc de produits de nature très différente, dans une seule unité de méthanisation se révèle déficiente, voire inefficiente, car la flore bactérienne, présente dans l'unité unique de méthanisation, risque de présenter des difficultés à s'adapter et se spécialiser convenablement pour les trois familles de produits différentes.

En résumé, l'efficacité d'une unité réalisant à la fois la méthanisation de biomasses ordinaires, la méthanisation des huiles de pyrogazéification et la biométhanation du syngaz s'avère être limitée.

Plus largement, les différents procédés de méthanation proposés ou mis en œuvre à l'heure actuelle pour valoriser les gaz de pyrogazéification en les transformant partiellement ou totalement en méthane présentent un grand nombre de difficultés.

Plus largement encore, les différents procédés proposés ou mis en œuvre à l'heure actuelle pour valoriser les gaz de pyrogazéification sous forme de chaleur et/ou d'électricité, et/ou en les transformant partiellement ou totalement en méthane, présentent un grand nombre de difficultés.

Au vu de ce qui précède, l'invention a notamment pour objet de proposer un dispositif capable de transformer efficacement des matières organiques en gaz constitués principalement de méthane et/ou d'hydrogène et/ou de dioxyde de carbone par couplage de procédés thermochimiques et biologiques, en minimisant les coûts d'investissement et d'exploitation mis en œuvre pour réaliser une telle transformation, et ceci tout en assurant une bonne fiabilité du dispositif, notamment en minimisant l'installation de dispositifs coûteux de lavage des gaz de pyrogazéification et en évitant l'affaiblissement et la dégradation de la flore bactérienne et en assurant sa bonne qualité dans le temps.

La présente invention a notamment pour objet un dispositif de transformation de matières organiques solides en mélanges de méthane (CH₄) et/ou d'hydrogène (H₂) et/ou de dioxyde de carbone (CO₂) comprenant au moins cinq fonctions :
(i) une fonction de pyrogazéification des matières organiques solides afin de former un gaz de pyrogazéification,
(ii) une fonction de vaporeformage des huiles de pyrogazéification contenues dans le gaz de pyrogazéification issu de la fonction (i),
(iii) une fonction de réaction de gaz à l'eau appliquée aux gaz issus de la fonction (ii),
(iv) une fonction de refroidissement et de lavage des gaz issus de la fonction (iii),
(v) une fonction de biométhanation des gaz issus de la fonction (iv).

En d'autres termes, le dispositif selon l'invention présente en série une fonction de pyrogazéification, une fonction de reformage à la vapeur, une fonction de réaction de gaz à l'eau, une fonction de refroidissement et de lavage et une fonction de méthanation par voie biologique ou biométhanation afin de transformer des matières organiques solides, tels que définis précédemment, en mélanges de méthane et/ou d'hydrogène et/ou de dioxyde de carbone. Ainsi le dispositif selon l'invention permet de transformer efficacement les matières organiques solides en méthane et/ou d'hydrogène et/ou de dioxyde de carbone, préférentiellement du biogaz, en minimisant les coûts d'investissement et d'exploitation, notamment liés aux équipements destinés au lavage les gaz de pyrogazéification et au traitement des huiles de pyrogazéification, tout en présentant un fonctionnement fiable capable de réduire les risques d'affaiblissement et de dégradation de la flore bactérienne utilisée dans l'obtention du biogaz.

En particulier, le dispositif selon l'invention permet de traiter efficacement les gaz de pyrogazéification, produits à partir de matières organiques solides, en diminuant leur caractère encrassant via une fonction de vaporeformage des huiles de pyrogazéification contenues dans lesdits gaz ainsi que leur caractère toxique via une fonction de réaction de gaz à l'eau.

Ainsi le dispositif selon l'invention présente l'avantage de valoriser les huiles de pyrogazéification et les gaz toxiques, notamment le monoxyde de carbone, contenus dans les gaz de pyro gazéification.

D'une manière générale, le dispositif selon l'invention présente l'avantage d'être fiable et robuste car les différentes fonctions minimisent les risques de condensation des huiles de pyrogazéification tout au long du processus de transformation des matières organiques solides.

La fonction de pyrogazéification a pour effet de transformer les matières organiques solides en char, d'une part, et en gaz de pyrogazéification, d'autre part, qui sont principalement composés d'un mélange de gaz incondensables et de gaz condensables, lesquels gaz condensables sont principalement composés d'huiles de pyrogazéification et de vapeur d'eau.

Préférentiellement, la fonction de pyrogazéification des matières organiques solides est exercée par une unité de pyro gazéification.

L'unité de pyrogazéification peut correspondre à n'importe quel dispositif dans lequel une réaction de pyrogazéification des matières organiques solides est mise en oeuvre.

De préférence, la fonction pyrogazéification est réalisée sans injection d'air afin de ne pas introduire d'azote dans le dispositif. En effet, l'introduction d'azote dans le dispositif a pour conséquence de produire des gaz de pyrogazéification contenant de l'azote. Autrement dit, l'azote se retrouverait en mélange avec le gaz de pyrogazéification produit par le dispositif.

En d'autres termes, en cas d'introduction d'air dans la fonction de pyrogazéification, le gaz produit par le dispositif est susceptible d'être principalement constitué d'un mélange de méthane (CH₄), monoxyde de carbone (CO), dioxyde de carbone (CO₂) et de diazote (N₂). Or le méthane (CH₄) et le diazote (N₂) présentent l'inconvénient d'être des gaz très difficiles à séparer l'un de l'autre de façon économiquement supportable, ce qui rend par la suite le biogaz obtenu difficile à épurer de façon suffisante pour conduire à un biométhane suffisamment pur pour être injectable dans les réseaux de gaz.

La fonction de vaporeformage (ou reformage à la vapeur d'eau) des huiles de pyrogazéification contenues dans le gaz de pyrogazéification produit par la fonction (i), est de préférence exercée par une unité de vaporeformage dédiée.

Le vaporeformage des huiles a pour fonction principale de transformer les huiles de pyrogazéification contenues dans les gaz de pyrogazéification en un mélange de gaz incondensables, essentiellement composé d'hydrogène (H₂) et de monoxyde de carbone (CO).

De manière générale, le vaporeformage des huiles de pyrogazéification est réalisé en introduisant des huiles à l'état liquide et de la vapeur d'eau dans un réacteur à moyenne température en présence de catalyseurs. Dans ces procédés de vaporeformage catalytique des huiles, la température du réacteur est généralement comprise entre 260 et 700°C, et la pression généralement comprise entre 15 et 35 bars.

La présence de catalyseurs dans un réacteur de vaporeformage peut parfois poser des problèmes de fiabilité dans la mesure où les catalyseurs sont souvent des matériaux fragiles susceptibles de perdre leurs propriétés en cas d'exploitation imparfaite du réacteur (variations de températures, présence d'impuretés). Dans la présente invention, le vaporeformage des huiles est de préférence réalisé selon un procédé non catalytique à haute température, c'est-à-dire en l'absence de catalyseurs susceptibles de déclencher ou faciliter la réaction de vaporeformage.

Les huiles de pyrogazéification contenues dans les gaz de pyrogazéification sont traitées par la fonction de vaporeformage (ii) en présence d'eau à des températures pouvant aller de 1000 et 1400°C.

Plus précisément, les huiles de pyrogazéification sont introduites, en présence d'eau, dans un réacteur chauffé à une température allant de 1000 et 1400°C.

A cette température, le vaporeformage des huiles peut se dérouler sans la nécessité de disposer de catalyseurs capables de déclencher ou faciliter la réaction. Le temps de séjour peut être compris entre 2 et 10 secondes.

De préférence, la totalité des gaz de pyrogazéification issus de la fonction de pyrogazéification est introduite dans la fonction de vaporeformage sans refroidissement préalable ce qui signifie que les huiles de pyrogazéification, issues de la fonction de pyrogazéification, sont introduites sous forme vaporisée, en même temps que les gaz de pyro gazéification.

L'introduction des huiles de pyrogazéification à l'état gazeux présente un certain nombre d'avantages.

D'une part, les huiles de pyrogazéification sont plus facilement manipulables à l'état gazeux que lorsqu'elles sont condensées. En effet, à l'état condensé, les huiles présentent la plupart du temps une viscosité élevée et une grande hétérogénéité.

D'autre part, les huiles préchauffées et déjà vaporisées nécessitent moins d'énergie pour être chauffées, en particulier à des températures supérieures à 1000°C, que si elles avaient été introduites à l'état liquide à des températures moins élevées. Ainsi l'énergie requise pour chauffer les huiles de pyrogazéification à des températures supérieures à 1000°C est plus faible pour des huiles, se trouvant déjà vaporisées en sortie de la fonction (i), que pour des huiles condensées.

Enfin, l'introduction d'huiles chaudes (à des températures supérieures à leur température de condensation) et à l'état gazeux dans la fonction de vaporeformage évite le recours à des équipements coûteux et complexes de refroidissement des gaz de pyrogazéification et de condensation des huiles de pyrogazéification.

En d'autres termes, la fonction de vaporeformage permet d'éviter le recours à des équipements coûteux de refroidissement de lavage des gaz de pyrogazéification permettant d'éliminer les huiles de pyrogazéification.

L'eau permettant de réaliser la réaction de vaporeformage des huiles de pyrogazéification dans la fonction de vaporeformage peut être introduite, soit principalement sous forme de vapeur directement dans la fonction de vaporeformage, soit, de préférence, principalement sous forme liquide ou sous forme de vapeur dans la fonction de pyrogazéification, soit partiellement et à la fois dans les deux modes de réalisation mentionnées.

De préférence, la fonction de pyrogazéification est mise en œuvre en présence d'eau.

En d'autres termes, l'eau est introduite sous forme liquide ou de vapeur d'eau dans la fonction de pyrogazéification.

De cette manière, la vapeur d'eau, issue de la fonction de pyrogazéification et contenue dans les gaz de pyrogazéification, est introduite dans la fonction de vaporeformage en même temps que les huiles de pyrogazéification et les gaz de pyrogazéification.

Autrement dit, l'eau permettant la réaction de vaporeformage est de préférence introduite dans la fonction de vaporeformage (ii) sous forme de vapeur en mélange avec les huiles de pyrogazéification vaporisées présentes dans les gaz de pyrogazéification.

De manière avantageuse, la fonction de vaporeformage (ii) permet d'éviter de mettre en œuvre une digestion anaérobie des huiles de pyrogazéification qui aurait pour but de diminuer le caractère encrassant du gaz de pyrogazéification.

Grâce à la fonction de vaporeformage, les huiles de pyrogazéification sont transformées en un mélange de gaz incondensables principalement composés d'hydrogène (H₂) et de monoxyde de carbone (CO), lequel mélange de gaz incondensable est très énergétique et d'une manipulation, donc d'une valorisation, plus aisée qu'un gaz contenant des huiles souvent visqueuses et hétérogènes rendant leur manipulation difficile.

De manière avantageuse, la fonction de vaporeformage permet la production d'un gaz ayant une teneur en hydrogène (H₂) sensiblement augmentée.

D'une façon générale, la fonction de vaporeformage présente l'avantage d'être une fonction simple, robuste et fiable, qui rend non nécessaire, d'une part, l'installation de tout équipement de lavage des gaz de pyrogazéification et, d'autre part, l'installation de tout type d'équipement de séparation et/ou de méthanisation des huiles de pyrogazéification. De tels équipements présentent généralement un coût d'investissement élevé et induisent une exploitation et une maintenance exigeantes et difficiles engendrant le plus souvent des problèmes de fiabilité et des coûts d'exploitation élevés.

Les fonctions de pyrogazéification et de vaporeformage des huiles de pyrogazéification peuvent être exercées dans un équipement unique.

Toutefois, de manière avantageuse les fonctions de pyrogazéification et de vaporeformage sont exercées dans deux équipements séparés.

En effet, la fonction de pyrogazéification est généralement exercée à une température moyenne qui est inférieure à la température de vaporisation de nombreux éléments potentiellement contaminants (métaux lourds, ...). Dans ces conditions, les matières potentiellement contaminantes non vaporisées se retrouvent ainsi en mélange avec le char, produit dans la fonction de pyrogazéification, et ne se trouvent ni dans les gaz de pyrogazéification, ni dans le biogaz produit par la fonction de biométhanation (pas de contamination atmosphérique), ni dans les digestats produits par la fonction biométhanation (pas de contamination des effluents liquides).

La fonction de réaction de gaz à l'eau (plus connue sous son nom anglais de Water Gas Shift réaction, ou encore WGS) des gaz issus de la fonction de vaporeformage (ii) est exercée de préférence par une unité de réaction de gaz à l'eau.

L'unité de gaz à l'eau peut correspondre à n'importe quel dispositif dans lequel une réaction de gaz à l'eau des gaz issus de la fonction de vaporeformage (ii), est mise en oeuvre.

La fonction de réaction de gaz à l'eau a pour effet principal de transformer tout ou partie du monoxyde de carbone contenu dans le mélange des gaz, issus de la fonction de vaporeformage, en un mélange d'hydrogène (H₂) et de dioxyde de carbone (CO₂) selon la formule :

CO + H₂O <-> H₂ + CO₂

La fonction de réaction de gaz à l'eau présente ainsi l'avantage de diminuer la teneur en monoxyde de carbone en le transformant en un mélange constitué d'hydrogène (H₂) et de dioxyde de carbone (CO₂) ce qui diminue le caractère toxique du gaz traité ultérieurement par la fonction de biométhanation.

En effet, le monoxyde de carbone, au dessus d'une certaine concentration de dilution, peut s'avérer être toxique pour les bactéries présentes dans le la fonction de biométhanation. Ainsi la fonction de réaction de gaz à l'eau permet la production d'un mélange gazeux majoritairement composé d'hydrogène (H₂) et de dioxyde de carbone (CO₂), ne contenant que très peu, voire pas du tout, de monoxyde de carbone, réduisant ainsi son caractère toxique vis-à-vis des bactéries présentes dans la fonction de biométhanation.

La fonction (iii) permet donc de réduire la teneur en monoxyde de carbone qui est un gaz toxique dont la manipulation est dangereuse et strictement réglementée.

De manière avantageuse, la fonction (iii) permet d'augmenter la teneur en hydrogène dans le gaz issu de la fonction de vaporeformage (ii).

Ainsi la fonction (iii) permet une meilleure valorisation des gaz issus de la fonction de vaporeformage (ii) en transformant le monoxyde de carbone en hydrogène.

La réaction de gaz à l'eau est le plus souvent réalisée en mélangeant les gaz contenant du monoxyde de carbone et de la vapeur d'eau dans un réacteur à moyenne température en présence de catalyseurs. Dans ces procédés, la température du réacteur est généralement comprise entre 190 et 350°C.

Comme indiqué précédemment, la présence de catalyseurs dans un réacteur de gaz à l'eau peut parfois poser des problèmes de fiabilité dans la mesure où les catalyseurs peuvent être des matériaux fragiles susceptibles de perdre leurs propriétés en cas d'exploitation imparfaite du réacteur (variations de température, présence d'impuretés).

La réaction de gaz à l'eau peut aussi être réalisée selon un procédé non catalytique à haute température (une température supérieure à 350°C). En particulier, dans le cas d'une réaction de gaz à l'eau non catalytique, les gaz contenant le monoxyde de carbone, issus de la fonction (ii), sont introduits, en présence de vapeur d'eau, dans la fonction de réaction de gaz à l'eau, à une température supérieure à 350°C et pouvant aller jusqu'à 900°C.

A cette température, la réaction de gaz à l'eau peut se dérouler sans la nécessité d'ajouter des catalyseurs pour déclencher ou faciliter la réaction permettant ainsi de diminuer le coût lié à cette réaction.

De préférence, la totalité des gaz issus de la fonction de vaporeformage est introduite dans la fonction de réaction de gaz à l'eau.

Avant leur introduction dans la fonction de réaction de gaz à l'eau, les gaz issus de la fonction de vaporeformage sont de préférence refroidis jusqu'à la température de réaction mise en œuvre dans la fonction de réaction de gaz à l'eau. La réaction de gaz à l'eau étant exothermique, un refroidissement supplémentaire peut être envisagé. Ce refroidissement peut, par exemple, être réalisé par une injection additionnelle de vapeur d'eau dans la fonction de réaction de gaz à l'eau.

Ainsi le monoxyde de carbone est introduit dans la fonction de réaction de gaz à l'eau en même temps que les gaz produits par cette fonction.

L'eau nécessaire à la réaction de gaz à l'eau dans la fonction (iii) peut être introduite, soit sous forme de vapeur directement dans la fonction de réaction de gaz à l'eau, soit sous forme de vapeur directement dans la fonction de vaporeformage, soit, de préférence, sous forme liquide ou sous forme de vapeur dans la fonction de pyrogazéification, soit partiellement et à la fois dans les modes de réalisation précédemment mentionnées.

De préférence, la fonction de pyrogazéification est mise en œuvre en présence d'eau.

De cette manière, la vapeur d'eau, issue de la fonction de pyrogazéification et contenue dans les gaz de pyrogazéification produits, est introduite dans la fonction de vaporeformage, en même temps que les huiles de pyrogazéification et les gaz de pyrogazéification, puis dans la fonction de réaction de gaz à l'eau.

En particulier, l'eau, utilisée dans la fonction de réaction de gaz à l'eau, correspond à l'eau n'ayant pas réagi avec les huiles de pyrogazéification dans la fonction de pyrogazéification ni dans la fonction de vaporeformage.

Dans ce dernier cas, l'eau permettant la réalisation de la réaction de gaz à l'eau est introduite sous forme de vapeur et est contenue dans les gaz issus de la fonction de vaporeformage.

Autrement dit, l'eau permettant la réaction de gaz à l'eau du monoxyde de carbone est de préférence introduite sous forme de vapeur dans la fonction de réaction de gaz à l'eau en mélange avec le monoxyde de carbone présent dans les gaz issus de la fonction de vaporeformage.

D'une façon générale, la fonction de réaction de gaz à l'eau présente l'avantage d'être une fonction simple et fiable, qui produit un mélange gazeux majoritairement composé d'hydrogène (H₂) et de dioxyde de carbone (CO₂), dont la valorisation énergétique est plus simple que celle des mélanges de gaz plus complexes.

Les fonctions de vaporeformage des huiles de pyrogazéification et de gaz à l'eau peuvent être exercées dans un équipement unique.

Toutefois, de manière avantageuse, les fonctions de vaporeformage des huiles de pyrogazéification et de gaz à l'eau sont exercées dans deux équipements séparés.

La fonction de refroidissement et de lavage a lieu de préférence dans une unité de refroidissement et de lavage dédiée.

Elle a pour effet premier de refroidir les gaz issus de la fonction de réaction de gaz à l'eau.

Il en résulte, premièrement, une condensation des huiles éventuellement encore présentes dans les gaz issus de la fonction (iii), qui n'ont pas subi de vaporeformage dans la fonction (ii). Ces huiles sont dites huiles réfractaires et sont généralement présentes en quantité très faible ou à l'état de traces, voire totalement absentes dans les gaz issus de la fonction (ii) puis (iii).

Ainsi le dispositif selon l'invention permet non seulement de minimiser le plus possible la fraction d'huiles de pyrogazéification dans les gaz de pyrogazéification mais également de les maintenir à une température supérieure à leur température de condensation de manière à éviter leur condensation dans les différentes fonctions (i) à (iii) et les éventuels équipements et/ou unités se trouvant en amont de la fonction de refroidissement et/ou de lavage.

La condensation des huiles de pyrogazéification réfractaires, intervenant uniquement dans la fonction de refroidissement et/ou de lavage, dans de très faibles quantités, permet d'améliorer la fiabilité, la robustesse et la durée de vie du dispositif selon l'invention ainsi que la qualité du ou des mélanges de méthane (CH₄) et/ou d'hydrogène (H₂) et/ou de dioxyde de carbone (CO₂) produit(s).

Il en résulte, deuxièmement, une condensation de l'eau vaporisée présente en excès dans les gaz issus de la fonction de réaction de gaz l'eau, laquelle eau vaporisée en excès est constituée de la fraction de l'eau qui n'a pas réagi avec les matières organiques dans la fonction (i), et qui n'a pas réagi avec les huiles de pyrogazéification dans la fonction (ii), et qui n'a pas réagi avec le monoxyde de carbone dans la fonction (iii).

Il en résulte, enfin, un entrainement dans la fraction liquide, des condensats des éventuels autres gaz condensables et des éventuelles particules fines solides (char, cendres, particules minérales etc.) éventuellement présentes dans les gaz issus de la fonction (iii).

Finalement, la fonction de refroidissement et de lavage a pour effet principal de laver les gaz issus de la fonction (iii). Les produits en sortie de la fonction (iv) sont composés, d'une part, d'un mélange de gaz incondensables, majoritairement constitués d'hydrogène (H₂) et de dioxyde de carbone (CO₂), et, d'autre part, d'un liquide composé principalement d'eau et éventuellement de traces d'huiles réfractaires et de faibles concentrations de matières solides en suspension qui ont été entrainées dans les gaz de pyrogazéification.

La fonction de refroidissement et de lavage permet également de favoriser la qualité du biogaz produit dans la fonction de biométhanation en minimisant les risques d'introduction des huiles de pyrogazéification réfractaires dans la fonction de biométhanation, ce qui pourrait conduire à une altération de la qualité des bactéries et une inhibition de leur activité.

De manière avantageuse, une quantité de liquide additionnelle, dit liquide de lavage, peut être ajoutée dans le liquide susceptible d'être utilisé dans la fonction (iv) afin de favoriser la fonction de lavage des gaz issus de la fonction de gaz à l'eau. De préférence, ce liquide de lavage est de l'eau.

Avantageusement, la fonction (iv) de refroidissement et lavage peut être complétée par une fonction d'aspiration des gaz ayant pour objet principal la création d'une légère dépression dans les unités dans lesquelles sont exercées les fonctions (i) de pyrogazéification et (ii) de vaporeformage et (iii) de réaction de gaz à l'eau et (iv) de refroidissement et lavage, de telle sorte que si l'étanchéité de l'une ou plusieurs de ces unités était imparfaite, toute fuite de gaz subséquente se ferait de l'extérieur vers l'intérieur et aucune émission vers l'extérieur de gaz toxique, notamment du monoxyde de carbone, ne pourrait se produire.

La fonction de biométhanation (v) a lieu de préférence dans une unité de méthanation dédiée.

Conformément à la présente invention, le gaz issu de la fonction (iv) et introduit dans la fonction de biométhanation est un mélange de gaz incondensables, majoritairement constitué d'hydrogène (H₂) et de dioxyde de carbone (CO₂). Il en résulte que la réaction qui s'applique majoritairement dans la fonction de biométhanation est :

CO₂ + 4H₂ → CH₄ + 2H₂O

De façon minoritaire, en présence de monoxyde de carbone (CO) n'ayant pas été transformé en hydrogène (H₂) et en dioxyde de carbone (CO₂) dans la fonction de gaz à l'eau, une réaction qui peut s'appliquer de façon minoritaire dans la fonction de biométhanation est la suivante :

CO + 3H₂ → CH₄ + H₂O

Conformément à la réaction majoritaire CO₂ + 4H₂ → CH₄ + 2H₂O, il faudrait en volume 4 fois plus d'hydrogène (H₂) que de dioxyde de carbone (CO₂) pour que ces gaz soient présents en quantités stœchiométriques. En l'espèce, la quantité de dioxyde de carbone (CO₂) est presque toujours en excès dans les gaz issus de la décomposition des matières organiques, et la fonction de biométhanation produit généralement un biogaz composé principalement de méthane (CH₄) et de dioxyde de carbone (CO₂).

Par ailleurs, les fonctions (ii) et (iii) permettent d'augmenter la teneur en hydrogène ce qui améliore le rapport entre les quantités d'hydrogène et de dioxyde de carbone mises en jeu dans la réaction de biométhanation.

Selon un mode de réalisation, le dispositif selon l'invention peur comprendre en outre une fonction de production de digestat liquide qui a pour objet principal, d'une part, la production de digestat liquide de qualité susceptible d'alimenter la fonction (v) de méthanation afin de générer une production abondante de digestat liquide contenant notamment une grande quantité de bactéries variées, de bonne qualité et en bonne santé, susceptibles de favoriser l'accomplissement de la fonction de biométhanation, et, d'autre part, d'alimenter la fonction (v) de méthanation en digestat liquide en quantité suffisante pour chacune des substances toxiques (monoxyde de carbone, huiles réfractaires etc.) éventuellement contenues dans le gaz issu de la fonction (iv) de refroidissement et lavage soit diluées dans le digestat liquide et se retrouve à une concentration inférieure à son seuil de toxicité pour les bactéries présentes dans le digestat liquide.

Selon un mode de réalisation avantageux, le dispositif selon l'invention comprend en outre une fonction de gazéification à la vapeur des chars issus de la fonction de pyrogazéification.

La fonction de gazéification des chars permet avantageusement de transformer les chars issus de la fonction de pyrogazéification en un mélange de gaz incondensables essentiellement composé d'hydrogène (H₂) et de monoxyde de carbone (CO).

La fonction de gazéification est mise en œuvre en présence de vapeur d'eau, notamment à des températures pouvant varier de 700 à 1400°C, de préférence à environ 800°C.

La fonction de gazéification des chars permet ainsi de valoriser avantageusement le charbon issu de la réaction de pyrogazéification.

Selon un mode de réalisation avantageux, le dispositif selon l'invention comprend en outre une fonction de séparation de l'hydrogène et des gaz issus de la fonction de refroidissement et de lavage afin de récupérer de l'hydrogène.

L'hydrogène se retrouve ainsi séparé des autres gaz incondensables issus de la fonction de refroidissement et de lavage.

La fonction de séparation de l'hydrogène (H₂), situé en aval de la fonction refroidissement et de lavage, et en dérivation, c'est-à-dire en parallèle de la fonction de biométhanation, permet de récupérer de l'hydrogène de manière à pouvoir l'utiliser dans de nombreuses applications, notamment dans des piles à combustible.

Selon encore un autre mode de réalisation avantageux, le dispositif comprend en outre une fonction de séparation des gaz issus de la fonction de biométhanation afin de récupérer du méthane.

Ainsi la fonction de séparation, situé en aval de la fonction de biométhanation, permet de séparer le méthane du dioxyde de carbone, et éventuellement de séparer aussi le méthane des autres gaz incondensables contenus minoritairement dans le biogaz produit par la fonction de biométhanation, de manière à pouvoir utiliser le méthane dans les applications souhaitées, par exemple en l'injectant dans un réseau de gaz naturel, ou comme carburant pour véhicules roulant au gaz naturel.

De préférence, la fonction de biométhanation est accomplie par une sous-unité de biométhanation constituée d'un ou plusieurs digesteurs anaérobies.

De préférence, le dispositif comprend en outre un circuit susceptible d'acheminer les condensats issus de la fonction de refroidissement et de lavage, en particulier l'eau et les éventuelles huiles réfractaires, en amont et/ou dans la fonction de pyrogazéification (i), de préférence dans la fonction de pyrogazéification (i).

Un tel circuit présente l'avantage de réaliser une revalorisation des huiles de pyrogazéification réfractaires et une réutilisation de l'eau dans les différentes fonctions du dispositif.

De préférence, le dispositif comprend en outre un circuit susceptible d'acheminer les condensats issus de la fonction de refroidissement et de lavage, en particulier l'eau et les éventuelles huiles réfractaires, en amont et/ou dans la fonction de vaporeformage (ii), de préférence dans la fonction de vaporeformage.

Un tel circuit présente l'avantage de réaliser une revalorisation des huiles de pyrogazéification réfractaires. En effet, les huiles véritablement réfractaires à une fonction de vaporeformage à haute température sont généralement en quantité infime ou nulle. Les huiles qui se retrouvent présentes dans la fonction de refroidissement et de lavage, et que l'on nomme ici huiles réfractaires, sont le plus souvent constituées de la fraction des huiles de pyrolyse qui n'a pas eu le temps de réagir dans la fonction de vaporeformage, au sein de laquelle les turbulences gazeuses ont été imparfaitement maitrisées, et qu'en conséquence une partie de ces huiles n'a pas eu le temps de réagir soit parce que leur temps de séjour a été trop court, soit parce que la température atteinte a été insuffisante. Un deuxième passage de ces huiles, dites réfractaires, dans la fonction de vaporeformage permettra dans la grande majorité des cas de les faire réagir et de finalement parvenir à transformer ces huiles en un mélange principalement constitué de l'hydrogène (H₂) et du monoxyde de carbone (CO).

De préférence, le dispositif comprend en outre un circuit susceptible d'acheminer les condensats issus de la fonction de refroidissement et de lavage, en particulier l'eau et éventuellement les huiles réfractaires, notamment l'eau, en amont et/ou dans la fonction de réaction de gaz à l'eau (iii), de préférence dans la réaction de gaz à l'eau. De préférence, l'acheminement des condensats issus de la fonction de refroidissement et de lavage (iv) dans la fonction de réaction de gaz à l'eau (iii) est réalisé quand lesdits condensats contiennent peu d'huiles réfractaires, i.e. comprennent majoritairement de l'eau, car la fonction de réaction de gaz à l'eau (iii) ne permet pas de les transformer en gaz.

Plus préférentiellement, le circuit est susceptible d'acheminer les condensats dans la fonction de pyrogazéification (i), et/ou la fonction de reformage à la vapeur (ii), et/ou dans la fonction de réaction de gaz à l'eau (iii).

Un tel circuit présente l'avantage de réaliser une revalorisation des huiles de pyrogazéification réfractaires si celles-ci sont réintroduites dans les fonctions de pyrogazéification ou de vaporeformage.

Selon un mode de réalisation, les fonctions de pyrogazéification (i), de reformage à la vapeur (ii), et/ou de fonction de réaction de gaz à l'eau (iii) peuvent être mises en œuvre dans le même équipement ou dans des équipements séparés.

En effet, les fonctions de pyrogazéification (i), de reformage à la vapeur (ii), et/ou de fonction de réaction de gaz à l'eau (iii) peuvent être mises en œuvre dans la même unité, dans deux unités distinctes ou peuvent être mises en œuvre chacune dans des unités distinctes.

De préférence, les fonctions de pyrogazéification (i), de reformage à la vapeur (ii) et fonction de réaction de gaz à l'eau (iii) sont réalisées dans trois unités distinctes.

Avantageusement, la fonction de refroidissement et de lavage RL est réalisée au moyen d'un ou plusieurs liquides de lavage sélectionnés parmi l'eau ou tout autre solvant organique des goudrons, c'est-à-dire toute matière organique susceptible de diluer les goudrons et leurs mélanges, et donc susceptibles d'extraire les goudrons du gaz issu de la fonction (iii).

Selon un mode de réalisation avantageux, la fonction de refroidissement et de lavage RL est réalisée au moyen d'un liquide de lavage composé principalement d'eau et d'un solvant organique des goudrons, par exemple le biodiesel ou l'huile de colza.

Selon un autre mode de réalisation avantageux, la fonction de refroidissement et de lavage RL est réalisée en deux étapes : une étape de refroidissement et lavage à l'eau et une étape de lavage au moyen d'un ou plusieurs liquides de lavage sélectionnés parmi les solvants organiques des goudrons, c'est-à-dire toute matière organique susceptible de diluer les goudrons et leurs mélanges, et donc susceptible de séparer et d'extraire les goudrons du gaz de pyro gazéification.

De préférence, le liquide de lavage de la première étape est l'eau, tandis que le liquide de lavage de la deuxième étape est un solvant organique des goudrons, par exemple le biodiesel ou l'huile de colza.

Par solvant organique des goudrons, on entend toute matière organique susceptible de diluer les goudrons et leurs mélanges dans des conditions de température à 25°C et à pression atmosphérique.

La présente invention concerne également un procédé de transformation de matières organiques solides en mélanges de méthane (CH₄) et/ou d'hydrogène (H₂) et/ou de dioxyde de carbone (CO₂) comprenant les étapes successives suivantes :
- une étape d'alimentation du dispositif selon l'invention tel que défini précédemment, par des matières organiques solides,
- une étape de pyrogazéification des matières organiques solides, réalisée par la fonction de pyrogazéification (i), pour former un gaz de pyrogazéification,
- une étape de reformage à la vapeur, réalisée par la fonction de vaporeformage (ii), des huiles de pyrogazéification contenues dans le gaz de pyrogazéification en présence d'eau,
- une étape de réaction de gaz à l'eau, réalisée par la fonction de réaction de gaz à l'eau (iii), des gaz issu de l'étape de reformage à la vapeur, en particulier du monoxyde de carbone, en présence d'eau,
- une étape de refroidissement et de lavage, réalisée par la fonction de refroidissement et de lavage (iv), des gaz issus de l'étape de réaction de gaz à l'eau,
- une étape de biométhanation, réalisée par la fonction de biométhanation (v), des gaz provenant de l'étape de refroidissement et de lavage.

Le procédé selon l'invention permet de transformer efficacement les matières organiques solides en mélanges de méthane (CH₄) et/ou d'hydrogène (H₂) et/ou de dioxyde de carbone (CO₂), en mettant successivement en œuvre les étapes précédemment décrites.

Le procédé peut comprendre une étape de gazéification à la vapeur des chars issus de la fonction de pyrogazéification, ce qui permet d'augmenter le rendement de transformation des matières organiques solides et d'obtenir une quantité plus importante de syngaz composé principalement de méthane (CH₄) et/ou d'hydrogène (H₂) et/ou de dioxyde de carbone (CO₂).

Le procédé peut comprendre, en aval de l'étape de refroidissement et de lavage, une étape de séparation de gaz visant à séparer l'hydrogène du le reste des gaz incondensables, en particulier du dioxyde de carbone, au sein des gaz issus de l'étape de refroidissement et de lavage.

Ainsi l'étape visant à séparer l'hydrogène du reste des gaz incondensables est mise en œuvre en parallèle de l'étape de biométhanation.

Cette étape permet de récupérer avantageusement l'hydrogène, notamment issu de l'étape de reformage à la vapeur au cours de laquelle le monoxyde de carbone est transformé en hydrogène et/ou de l'étape de réaction à gaz à l'eau, au cours de laquelle le dioxyde de carbone est transformé en hydrogène.

De préférence, le procédé comprend en outre une étape de séparation de gaz visant à séparer le méthane du reste des gaz, dont le dioxyde de carbone, au sein des biogaz issus de l'étape de biométhanation.

Conformément à cette étape, le méthane est séparé du dioxyde de carbone afin d'être utilisé dans les applications souhaitées, par exemple en l'injectant dans un réseau de gaz naturel, ou comme carburant pour véhicules roulant au gaz naturel.

De manière avantageuse, le procédé comprend en outre une étape de récupération des condensats, issus de l'étape de refroidissement et de lavage, pour être injectés avant ou au cours de l'étape de pyrogazéification, de préférence au cours de l'étape de pyro gazéification.

De manière avantageuse, le procédé comprend en outre une étape de récupération des condensats, issus de l'étape de refroidissement et de lavage, pour être injectés avant ou au cours de l'étape de vaporeformage, de préférence au cours de l'étape de vaporeformage.

De manière avantageuse, le procédé comprend en outre une étape de récupération des condensats, issus de l'étape de refroidissement et de lavage, pour être injectés avant ou au cours de l'étape de réaction de gaz à l'eau, de préférence au cours de l'étape de réaction de gaz à l'eau.

De manière avantageuse, une quantité additionnelle d'eau peut être ajoutée au cours de l'étape de refroidissement et de lavage, dans la fonction (iv).

De manière avantageuse, le procédé comprend en outre une étape de récupération de la chaleur issue de l'étape de refroidissement et de lavage pour être renvoyée avant ou au cours de l'étape de pyro gazéification.

De manière encore avantageuse, le procédé comprend en outre une étape de récupération de la chaleur issue de l'étape de refroidissement et de lavage pour être renvoyée avant ou au cours de l'étape de réaction de gaz à l'eau.

De manière avantageuse, le procédé comprend en outre une étape de récupération de la chaleur issue de l'étape de réaction de gaz à l'eau, ou bien issue de la réaction de vaporeformage, pour être renvoyée avant ou au cours de l'étape de pyrogazéification.

Ces étapes de récupération de chaleur et de renvoi de la chaleur récupérée dans une étape amont du procédé peut être mise en œuvre au moyen d'un ou plusieurs échangeurs de chaleur, lesquels peuvent par exemple récupérer la chaleur des gaz issus de la fonction (iii) de réaction de gaz à l'eau, ou bien des gaz issus de la fonction (ii) de vaporeformage, pour réchauffer les condensats issus de la fonction (iv) de refroidissement et lavage et les renvoyer dans un fonction amont du procédé ou bien pour réchauffer tout autre fluide caloporteur et le renvoyer dans un fonction amont du procédé.

De préférence, l'étape de pyrogazéification, l'étape de vaporeformage et l'étape de réaction de gaz à l'eau sont mises en œuvre dans la même unité du dispositif selon l'invention.

Avantageusement, le procédé selon l'invention comprend une étape de refroidissement et de lavage réalisée au moyen d'un ou plusieurs liquides de lavage sélectionnés parmi l'eau ou toute autre matière susceptible de diluer les goudrons et leurs mélanges issus de la fonction (iii).

De préférence, l'étape de refroidissement et de lavage est réalisée en deux étapes : une étape de refroidissement et de lavage à l'eau et une étape de lavage réalisée au moyen d'un ou plusieurs liquides de lavage sélectionnés parmi les solvants organiques des goudrons, i.e. toute matière organique susceptible de diluer les goudrons et leurs mélanges issus de la fonction (iii), et donc susceptible de séparer et d'extraire les goudrons du gaz de pyro gazéification.

D'autres avantages et caractéristiques de l'invention apparaîtront à l'examen de la description détaillée d'un mode de réalisation de l'invention nullement limitatif, et des dessins annexés, sur lesquels :

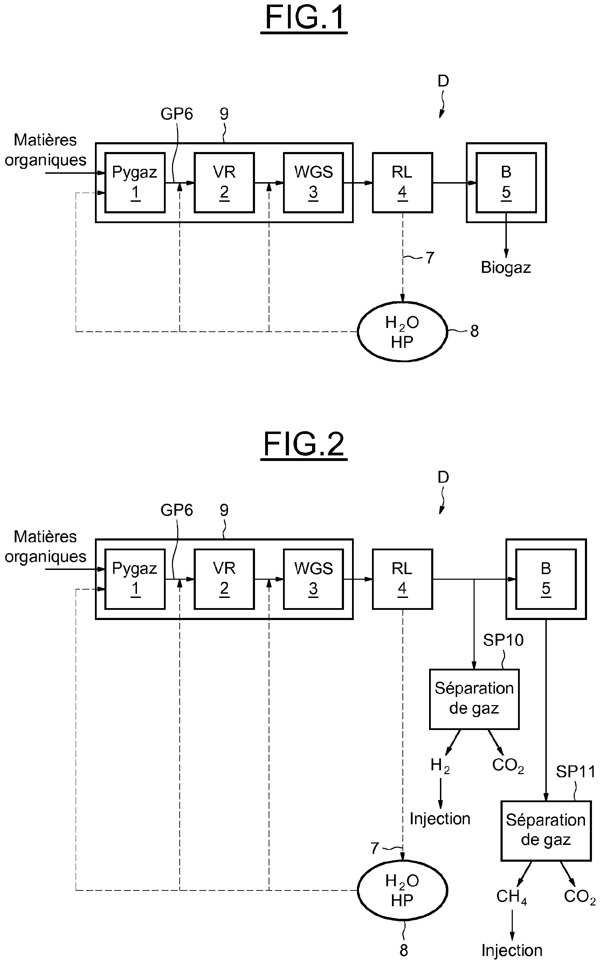

- la figure 1 représente une vue schématique d'un dispositif selon l'invention,
- la figure 2 représente une vue schématique plus détaillée du dispositif selon l'invention qui comprend en outre un système de séparation de gaz situé en aval de l'étape de refroidissement et de lavage, et en dérivation, c'est-à-dire en parallèle de l'étape de biométhanation, afin de séparer l'hydrogène du reste des gaz incondensables pour récupérer de l'hydrogène pur, et/ou un autre système de séparation de gaz situé en aval de la fonction de biométhanation afin de séparer le méthane (CH₄) du reste des gaz incondensables pour récupérer du méthane pur.

Sur la figure 1 est représenté de manière schématique un dispositif D, destiné à transformer des matières organiques solides en mélanges de méthane (CH₄) et/ou d'hydrogène (H₂) et/ou de dioxyde de carbone (CO₂), comprenant une fonction de pyrogazéification 1, dénommé Pygaz 1, une fonction de vaporeformage 2, dénommé VR 2, une fonction de réaction de gaz à l'eau 3, dénommé WGS 3, une fonction de refroidissement et de lavage 4, dénommé RL 4, et une fonction de biométhanation 5, dénommé B 5.

La fonction Pygaz 1 met en œuvre une réaction de pyrogazéification des matières organiques solides pour produire des gaz de pyrogazéification 6, dénommé GP 6.

De préférence, la fonction Pygaz 1 est exercée par une unité de pyrogazéification. Cette unité de pyrogazéification est de préférence un four tournant.

Les matières organiques solides susceptibles d'être traitées par la fonction Pygaz 1 peuvent être de natures très diverses.

Elles correspondent en général à des matières d'origine organique non fossiles, transformées ou non, ou bien à des matières organiques dérivées du pétrole. Parmi les grandes familles de matières d'origine organique non fossiles opportunément valorisables par pyrogazéification, on peut citer principalement, et de manière non exhaustive, les biomasses végétales d'origine agricole (le bois, les pailles, les menu-pailles et autres résidus de culture, les cultures énergétiques etc.), les déchets industriels banals non inertes et les déchets issus d'une collecte sélective de matières organiques d'origine municipale, (déchets de bois, papiers, cartons, textiles, etc.) ou encore les déchets d'activités de d'épuration (boues de stations d'épuration, etc.). Les matières dérivées du pétrole valorisables sont principalement des déchets d'objets en matière plastiques (résidus urbains d'objets en plastique de la vie courante, sac et autres emballages plastique, pneus, résidus de décharges automobiles etc.).

La réaction de pyrogazéification mise en œuvre par la fonction Pygaz 1 peut se dérouler en présence d'une quantité réduite ou en l'absence d'oxygène à des températures élevées, pouvant aller de 300 à 900°C au sein de l'unité de pyrogazéification afin de transformer les matières organiques en gaz de pyrogazéification GP 6.

La fonction VR 2 met en œuvre une réaction de vaporeformage des huiles de pyrogazéification, contenues dans les gaz pyrogazéification GP 6, pour les transformer en gaz incondensables, constitués essentiellement de mélanges de monoxyde de carbone et d'hydrogène.

De préférence, la fonction VR 2 est exercée par une unité de vaporeformage. Cette unité de vaporeformage est de préférence un four tubulaire.

Préférentiellement, la réaction de vaporeformage des huiles de pyrogazéification peut se dérouler à des températures allant de 1000 à 1400°C, notamment à des températures allant de 1100 à 1300, et en particulier à une température proche de 1200°C, en présence d'eau.

Ainsi, de préférence, la fonction VR 2 ne contient pas de catalyseur.

La fonction VR 2 a donc pour but de valoriser les huiles de pyrogazéification contenues dans les gaz de pyrogazéification GP 6 en les transformant en gaz incondensables, notamment en mélanges de monoxyde de carbone et d'hydrogène.

En d'autres termes, la fonction VR 2 permet de minimiser, voire d'éliminer, les huiles de pyrogazéification contenues dans les gaz de pyrogazéification GP 6, ce qui permet de diminuer le caractère encrassant du gaz de pyrogazéification.

L'eau permettant de réaliser la réaction de vaporeformage des huiles de pyrogazéification peut être introduite, soit principalement sous forme de vapeur directement dans la fonction VR 2, soit, de préférence sous forme liquide ou sous forme de vapeur dans la fonction Pygaz 1, soit à la fois dans la fonction Pygaz 1 et dans la fonction VR 2.

De préférence, l'eau est introduite sous forme liquide dans la fonction Pygaz 1.

Ainsi, à l'issue de la fonction VR 2, l'ensemble des gaz de pyrogazéification GP 6, provenant de la fonction Pygaz 1, est essentiellement transformé en un mélange de gaz incondensables contenant majoritairement du monoxyde de carbone et de l'hydrogène.

La fonction WGS 3 met en œuvre une réaction de gaz à l'eau afin de transformer le monoxyde de carbone, en présence de vapeur d'eau, en un mélange majoritairement composé de dioxyde de carbone et d'hydrogène.

De préférence, la fonction WGS 3 est exercée dans une unité de gaz à l'eau.

Préférentiellement, la réaction de gaz à l'eau du monoxyde de carbone se déroule en présence de vapeur d'eau à des températures pouvant aller de 350 à 900°C, notamment à une température d'environ 800°C.

Ainsi la réaction de gaz à l'eau peut se dérouler en absence de catalyseurs.

Selon un autre mode de réalisation, la réaction de gaz à l'eau se déroule en présence de catalyseurs, notamment à des températures allant de 190 à 350°C.

La réaction de gaz à l'eau est le plus souvent réalisée en mélangeant les gaz contenant du monoxyde de carbone et de la vapeur d'eau dans un réacteur à moyenne température en présence de catalyseurs. Dans ces procédés, la température du réacteur est généralement comprise entre 190 et 350°C.

La réaction de gaz à l'eau peut aussi être réalisée selon un procédé non catalytique à haute température (une température supérieure à 350°C). En particulier, dans le cas d'une réaction de gaz à l'eau non catalytique, les gaz contenant le monoxyde de carbone, issus de la fonction (ii), sont introduits, en présence de vapeur d'eau, dans la fonction de réaction de gaz à l'eau, à une température pouvant aller de 350 à 900°C.

A cette température, la réaction de gaz à l'eau peut se dérouler sans la nécessité d'ajouter des catalyseurs pour déclencher ou faciliter la réaction permettant ainsi de diminuer les coûts liés à cette réaction.

La fonction WGS 3 permet de réduire la teneur en monoxyde de carbone contenue dans les gaz incondensables issus de la fonction VR 2 ce qui conduit à diminuer le caractère toxique des gaz issus de la fonction VR 2 et à enrichir en hydrogène le gaz ainsi produit.

L'eau permettant de réaliser la réaction de gaz à l'eau peut être introduite, soit sous forme de vapeur directement dans la fonction WGS 3, soit sous forme de vapeur directement dans la fonction VR 2, soit sous forme liquide ou sous forme de vapeur dans la fonction Pygaz 1, soit à la fois dans la fonction WGS 3, et/ou dans la fonction VR 2 et/ou dans la fonction Pygaz 1.

De préférence, l'eau est introduite majoritairement sous forme liquide dans la fonction Pygaz 1.

La fonction RL 4 permet de mettre en œuvre un refroidissement et un lavage des gaz incondensables issus de la fonction WGS 3.

De préférence, la fonction RL 4 est mise en œuvre par une unité contenant un liquide de refroidissement et de lavage, lequel peut être partiellement ou totalement constitué de vapeur d'eau issue de la fonction WGS 3 et condensée dans la fonction RL 4. Ainsi le refroidissement et le lavage sont préférentiellement mis en œuvre en injectant les gaz, issus de la fonction WGS 3, dans un liquide de lavage froid ou refroidi et/ou par aspersion desdits gaz par un tel liquide. Le liquide de lavage a notamment de préférence une température comprise entre 35 et 50°C.

Dans un premier temps, le liquide de lavage permet d'abaisser la température des gaz incondensables en sortie de la fonction WGS 3 et, dans un second temps, de condenser et séparer les gaz incondensables et les huiles de pyrogazéification récalcitrantes, c'est-à-dire la fraction des huiles issues de Pygaz 1 n'ayant pas réagi lors du vaporeformage mis en œuvre par la fonction VR 2.

Par ailleurs, le liquide de refroidissement et de lavage favorise également la condensation de la fraction de la vapeur d'eau n'ayant pas réagi avec le monoxyde de carbone lors de la réaction de gaz l'eau mise en œuvre par la fonction WGS 3.

Ainsi la fonction RL 4 permet de condenser la vapeur d'eau en excès en provenance de la fonction WGS 3 et de laver les gaz incondensables en entraînant les huiles de pyrogazéification non transformées dans la fonction VR 2.

De préférence, l'eau et les huiles de pyrogazéification non transformées, issues de la fonction RL 4 peuvent être récupérées et injectées en amont et/ou dans la fonction Pygaz 1 et/ou dans la fonction VR 2 et/ou dans la fonction WGS 3.

L'eau et les huiles de pyrogazéification non transformées, forment des condensats 8 issus de la fonction RL 4.

En d'autres termes, les condensats 8 issus de la fonction RL 4 peuvent être récupérés, traités et valorisés à différents endroits du dispositif 1 pour être transformés en gaz incondensables.

En particulier, les condensats 8 peuvent être mélangés aux matières organiques solides de manière à être traités par la fonction Pygaz 1.

Cette récupération peut être mise en œuvre par un circuit 7 représenté en pointillés sur la figure 1.

La fonction RL 4 permet de faciliter la biométhanation mise en œuvre ultérieurement par la fonction B 5 en améliorant la qualité du gaz à transformer par biométhanation et en contribuant à préserver l'activité et la qualité des bactéries.

De préférence, le liquide de refroidissement et de lavage peut être de l'eau, de préférence cette eau est partiellement ou totalement constituée de vapeur d'eau issue de la fonction WGS 3 et condensée dans la fonction RL 4.

Avantageusement, une quantité d'eau supplémentaire est ajoutée dans la fonction de refroidissement et de lavage afin d'assurer un meilleur lavage et une bonne dilution des condensats.

Cet ajout peut être effectué de manière continue au cours du fonctionnement du dispositif ou séquentiellement.

Le liquide de refroidissement et de lavage peut également être constitué en totalité ou en partie par du digestat liquide provenant d'une unité de production de digestat liquide non représentée sur la figure 1 et distincte du dispositif D.

De préférence, le liquide de refroidissement et de lavage peut être choisi parmi l'eau ou tout autre liquide susceptible de diluer les goudrons et leurs mélanges.

Par exemple, le biodiesel ou l'huile de colza permettent avantageusement de diluer les goudrons tertiaires susceptibles d'être présents dans le gaz issu de la fonction WGS 3, notamment les composés aromatiques cycliques tels que le benzène, le toluène, le naphtalène, le phénol, l'indène, ...

Avantageusement, la fonction RL 4 être complété par une fonction d'aspiration des gaz ayant pour objet principal la création d'une légère dépression dans les unités dans lesquelles sont exercées les fonctions Pygaz 1 et VR 2 et WGS 3 et RL 4, de telle sorte que si l'étanchéité de l'une ou plusieurs de ces unités était imparfaite, toute fuite de gaz subséquente se ferait de l'extérieur vers l'intérieur et aucune émission vers l'extérieur de gaz toxique, notamment du monoxyde de carbone, ne pourrait se produire.

La fonction B 5 met en œuvre une réaction de biométhanation des gaz incondensables issus de la fonction RL 4 pour les transformer en biogaz, majoritairement constitué de méthane et de dioxyde de carbone.

De préférence, la fonction B 5 est exercée par un digesteur anaérobie comprenant une cuve surmontée d'une membrane étanche constituant un espace de stockage de biogaz.

De manière alternative, la fonction B 5 peut être exercée par une sous-unité de biométhanation constitué d'un ou plusieurs digesteurs anaérobies.

Avantageusement, le ou les digesteur(s) anaérobie(s) dans le(s)quel(s) la fonction B 5 est exercée peut (peuvent) être couplé(s) à une unité de production de digestat liquide, non représentée sur la figure 1 et distincte du dispositif D, laquelle unité de production de digestat liquide a pour fonctions principales, d'une part, la production de digestat liquide de qualité susceptible d'alimenter la fonction B5 afin de générer une production abondante de digestat liquide contenant notamment une grande quantité de bactéries variées, de bonne qualité et en bonne santé, susceptibles de favoriser la fonction B 5, et, d'autre part, d'alimenter la fonction B 5 en digestat liquide en quantité suffisante pour chacune des substances toxiques (monoxyde de carbone, huiles réfractaires etc.) éventuellement contenues dans le gaz issu de la fonction RL 4 soit diluées dans le digestat liquide et se retrouve à une concentration inférieure à son seuil de toxicité pour les bactéries présentes dans le digestat liquide.

Conformément à la figure 1, la fonction Pygaz 1, la fonction VR 2 et la fonction WGS 3 peuvent être réalisées par la même unité 9.

En d'autres termes, le dispositif D selon l'invention peut comprendre une unité 9 susceptible de réaliser la fonction Pygaz 1, la fonction VR 2 et la fonction WGS 3, une unité de refroidissement et de lavage et une unité de biométhanation.

En variante, la fonction Pygaz 1 et la fonction VR 2 sont réalisées dans des unités distinctes.

De préférence, la fonction Pygaz 1, la fonction VR 2 et la fonction WGS 3 sont réalisées par des sous-unités distinctes.

La figure 2 représente le dispositif selon l'invention qui comprend, en outre, un élément de séparation de gaz, dénommé SP 10, situé en aval de la fonction RL 4 et/ou un élément de séparation de gaz SP 11, situé en aval de la fonction B 5.

L'élément de séparation SP 10, situé en aval de la fonction RL 4, permet de séparer l'hydrogène et le reste des gaz incondensables, dont principalement le dioxyde carbone, au sein des gaz incondensables issus de la fonction RL 4.

En particulier, l'élément de séparation SP 10 présente l'avantage de permettre la récupération de l'hydrogène pour pouvoir l'utiliser dans de nombreuses applications, par exemple les piles à combustible.

L'élément de séparation SP 11, situé en aval de la fonction B 5, permet de séparer le méthane et le reste des gaz incondensables, dont principalement le dioxyde carbone, au sein du biogaz produit par la réaction de biométhanation mis en œuvre par la fonction B 5.

En particulier, l'élément de séparation SP 11 présente l'avantage de permettre la récupération du biométhane susceptible d'être ensuite utilisé en injection dans un réseau de méthane, ou comme carburant dans des véhicule roulant au méthane.

Avantageusement, le dispositif D selon l'invention peut également comporter une fonction de gazéification des chars (non représentée sur les figures) destinée à transformer les chars issus de la fonction pyrogazéification 1 en un mélange de gaz incondensables composé essentiellement de monoxyde de carbone et d'hydrogène.

Le mélange de gaz incondensable ainsi obtenu peut être ensuite acheminé vers la fonction WGS 3 afin de transformer le monoxyde de carbone, en présence de vapeur d'eau, en un mélange composé d'une plus forte proportion de dioxyde de carbone et d'hydrogène.

Ainsi le dispositif D selon l'invention permet de transformer efficacement les matières organiques en mélanges de méthane (CH₄) et/ou d'hydrogène (H₂) et/ou de dioxyde de carbone (CO₂) tout en minimisant les coûts d'investissement et d'exploitation susceptibles d'être liés aux équipements utilisés pour laver les gaz de pyrogazéification et traiter les huiles de pyrogazéification, et en contribuant à préserver la qualité de la flore bactérienne responsable de la production de biogaz.

Le dispositif D selon l'invention est donc fiable et robuste du fait de la minimisation, voire de l'élimination, des teneurs en huiles de pyrogazéification et de monoxyde de carbone dans les gaz injectés dans l'unité B 5.

Le dispositif D permet donc de transformer les gaz de pyrogazéification de manière fiable et moins coûteuse afin de produire notamment des gaz enrichis en hydrogène et du biogaz de qualité.

De préférence, le dispositif D selon l'invention permet de transformer efficacement les matières organiques en biométhane.

L'exemple suivant est donné à titre illustratif de la présente invention.

### Exemple

L'exemple suivant porte sur un dispositif selon l'invention permettant de transformer des boues produites par des stations d'épuration et des déchets lignocellulosiques en un gaz riche en hydrogène (H₂), méthane (CH₄) et dioxyde de carbone (CO₂).

Des études expérimentales et théoriques ont été réalisées en laboratoire sur une installation pilote à lit fluidisé conforme à l'invention mettant en œuvre en série les fonctions suivantes :
(i) une fonction de pyrogazéification mettant en œuvre une réaction de pyrogazéification des boues d'épuration et des déchets lignocellulosiques à une température allant de 700 à 850°C,
(ii) une fonction de vaporeformage des huiles de pyrogazéification, contenues dans le gaz de pyrogazéification issu de la fonction (i) pour les transformer en gaz incondensables, constitués essentiellement de mélanges de monoxyde de carbone (CO) et d'hydrogène (H₂),
(iii) une fonction de réaction de gaz à l'eau appliquée au gaz issu de la fonction (ii) destinée à transformer le monoxyde de carbone (CO), en présence de vapeur d'eau, en un mélange majoritairement composé de dioxyde de carbone (CO₂) et d'hydrogène (H₂),
(iv) une fonction de refroidissement et lavage du gaz issu de la fonction (iii)

Le dispositif permet l'obtention de gaz convenant à la production du biométhane par biométhanation ou de l'hydrogène. Les gaz obtenus peuvent être riche en hydrogène (dans une teneur supérieure à 40%) avec des teneurs en hydrocarbures légers (CH₄ et C₂H₄, C₂H₆, C₂H₂) pouvant dépasser 15%.

Le taux de production de gaz, défini comme le volume de gaz incondensable produit dans les conditions normales par kg de matière organique, dépend de la composition de déchets. Sa valeur est comprise entre 0,8 à 1,8 Nm³/kg de matière organique.

La composition du gaz produit est fortement affectée par la nature de déchets et le ratio des débits massiques de vapeur d'eau et de biomasse sèche F_{vap/}F_{B.}

Dans les conditions opératoires testées le rapport H₂/CO est compris entre 2 et 4.

L'utilisation d'olivine comme média fluidisé favorise les réactions de craquage et reformage des huiles de pyrogazéification ainsi que la réaction de gaz à l'eau.

Cette technologie permet la réalisation des fonctions de pyrogazéification, de reformage à la vapeur, de réaction de gaz à l'eau dans le même réacteur.

## Revendications

1. Dispositif (D) de transformation de matières organiques solides en mélanges de méthane (CH₄) et/ou d'hydrogène (H₂) et/ou de dioxyde de carbone (CO₂) comprenant au moins les cinq fonctions suivantes :
(i) une fonction de pyrogazéification Pygaz (1) de matières organiques solides afin de former un gaz de pyrogazéification GP (6),
(ii) une fonction de reformage à la vapeur VR (2) des huiles de pyrogazéification contenues dans le gaz de pyrogazéification GP (6) issu de la fonction (i),
(iii) une fonction de réaction de gaz à l'eau WGS (3) appliquée aux gaz issus de la fonction (ii),
(iv) une fonction de refroidissement et de lavage RL (4) des gaz issus de la fonction (iii),
(v) une fonction de biométhanation B (5) des gaz issus de la fonction (iv).

2. Dispositif (D) selon la revendication 1, **caractérisé en ce qu'**il comprend en outre une fonction de gazéification à la vapeur des chars issus de la fonction (i).

3. Dispositif (D) selon la revendication 1 ou 2, **caractérisé en ce qu'**il comprend en outre une fonction de séparation SP (10) des gaz issus de la fonction (iv) afin de récupérer de l'hydrogène.

4. Dispositif (D) selon l'une quelconque des revendications précédentes, **caractérisé en ce qu'**il comprend en outre une fonction de séparation SP (11) des gaz issus de la fonction (v) afin de récupérer du méthane.

5. Dispositif (D) selon l'une quelconque des revendications précédentes, **caractérisé en ce que** la fonction de biométhanation B (5) est accomplie par une sous-unité de biométhanation constituée d'un ou plusieurs digesteurs anaérobies.

6. Dispositif (D) selon l'une quelconque des revendications précédentes, **caractérisé en ce qu'**il comprend en outre un circuit (7) susceptible d'acheminer les condensats (8) en amont et/ou dans la fonction pyrogazéification Pygaz (1).

7. Dispositif (D) selon l'une quelconque des revendications précédentes, **caractérisé en ce qu'**il comprend en outre un circuit (7) susceptible d'acheminer les condensats (8) en amont et/ou dans la fonction de vaporeformage VR (2).

8. Dispositif (D) selon l'une quelconque des revendications précédentes, **caractérisé en ce qu'**il comprend en outre un circuit (7) susceptible d'acheminer les condensats (8) en amont et/ou dans la fonction de réaction à gaz à l'eau WGS (4).

9. Dispositif (D) selon l'une quelconque des revendications précédentes, **caractérisé en ce que** la fonction de refroidissement et de lavage RL (4) est réalisée au moyen d'un liquide de lavage sélectionné parmi l'eau ou un solvant organique des goudrons.

10. Procédé de transformation de matières organiques solides en mélanges de méthane (CH₄) et/ou d'hydrogène (H₂) et/ou de dioxyde de carbone (CO₂) comprenant les étapes successives suivantes:
- une étape d'alimentation d'un dispositif (D), tel que défini selon l'une quelconque des revendications 1 à 9, par des matières organiques solides,
- une étape de pyrogazéification des matières organiques solides, réalisée par la fonction Pygaz (1), pour former un gaz de pyrogazéification GP (6),
- une étape de reformage à la vapeur, réalisée par la fonction VR (2), des huiles de pyrogazéification contenues dans le gaz de pyrogazéification GP (6) en présence d'eau,
- une étape de réaction de gaz à l'eau, réalisée par la fonction WGS (3), des gaz issus de l'étape de reformage à la vapeur en présence d'eau,
- une étape de refroidissement et de lavage, réalisée par la fonction RL (4), des gaz issus de l'étape de réaction de gaz à l'eau,
- une étape de biométhanation, réalisée par la fonction de biométhanation B (5), des gaz provenant de l'étape de refroidissement et de lavage.

11. Procédé selon la revendication 10, **caractérisé en ce qu'**il comprend une étape de gazéification à la vapeur des chars issus de la fonction Pygaz (1).

12. Procédé selon la revendication 10, **caractérisé en ce qu'**il comprend en outre, en aval de l'étape de refroidissement et de lavage, une étape visant à séparer l'hydrogène et le reste des gaz, dont le dioxyde de carbone, au sein des gaz issus de l'étape de refroidissement et de lavage.

13. Procédé selon la revendication 10, **caractérisé en ce qu'**il comprend en outre une étape de séparation visant à séparer le méthane et le reste des gaz, dont le dioxyde de carbone, au sein des biogaz issus de l'étape de biométhanation.

14. Procédé selon l'une quelconque des revendications 10 à 13, **caractérisé en ce qu'**il comprend en outre une étape de récupération des condensats (8) issus de l'étape de refroidissement et de lavage, pour être injectés avant ou au cours de l'étape de pyrogazéification.

15. Procédé selon l'une quelconque des revendications 10 à 14, **caractérisé en ce qu'**il comprend en outre une étape de récupération des condensats (8) issus de l'étape de refroidissement et de lavage, pour être injectés avant ou au cours de l'étape de reformage à la vapeur.

16. Procédé selon l'une quelconque des revendications 10 à 15, **caractérisé en ce qu'**il comprend en outre une étape de récupération des condensats (8) issus de l'étape de refroidissement et de lavage, pour être injectés avant ou au cours de l'étape de réaction de gaz à l'eau.

17. Procédé selon l'une quelconque des revendications 10 à 16, **caractérisé en ce qu'**il comprend en outre une étape de récupération de la chaleur issue de l'étape de refroidissement et de lavage, pour être renvoyée avant ou au cours de l'étape de pyrogazéification.

18. Procédé selon l'une quelconque des revendications 10 à 17, **caractérisé en ce qu'**il comprend en outre une étape de récupération de la chaleur issue de l'étape de refroidissement et de lavage, pour être renvoyée avant ou au cours de l'étape de réaction de gaz à l'eau.

19. Procédé selon l'une quelconque des revendications 10 à 18, **caractérisé en ce qu'**il comprend en outre une étape de récupération de la chaleur issue de l'étape de gaz à l'eau, pour être renvoyée avant ou au cours de l'étape de pyrogazéification.

20. Procédé selon l'une quelconque des revendications 10 à 19, caractérisé en ce l'étape de refroidissement et de lavage RL est réalisée au moyen d'un ou plusieurs liquides de lavage sélectionnés parmi l'eau ou un solvant organique des goudrons présents dans le gaz de pyrogazéification GP (6) issu de l'étape de pyrogazéification des matières organiques solides, réalisée par la fonction Pygaz (1),

21. Procédé selon l'une quelconque des revendications 10 à 20, caractérisé en ce l'étape de refroidissement et de lavage RL est réalisée en deux étapes : une étape de refroidissement et lavage à l'eau et une étape de lavage au moyen d'un ou plusieurs liquides de lavage sélectionnés parmi les solvants organiques des goudrons présents dans le gaz de pyrogazéification GP (6) issu de l'étape de pyrogazéification des matières organiques solides, réalisée par la fonction Pygaz (1).

## Patentansprüche

1. Vorrichtung (D) zur Umwandlung von organischen Feststoffen in Gemische aus Methan (CH₄) und/oder Wasserstoff (H₂) und/oder Kohlendioxid (CO₂) die mindestens die folgenden fünf Funktionen umfasst:
(i) eine Funktion Pygaz (1) zur Pyrovergasung von organischen Feststoffen, um ein Pyrovergasungsgas GP (6) zu bilden,
(ii) eine Funktion VR (2) zur Dampfreformierung der Pyrovergasungsöle, die in dem aus der Funktion (i) hervorgehenden Pyrovergasungsgas GP (6) enthalten sind,
(iii) eine Funktion WGS (3) zur Wassergas-Shift-Reaktion, die auf die aus der Funktion (ii) hervorgehenden Gase angewendet wird,
(iv) eine Funktion RL (4) zum Kühlen und Waschen der aus der Funktion (iii) hervorgehenden Gase,
(v) eine Funktion B (5) zur Biomethanisierung der aus der Funktion (iv) hervorgehenden Gase.

2. Vorrichtung (D) nach Anspruch 1, **dadurch gekennzeichnet, dass** sie weiter eine Funktion zur Dampfvergasung der aus der Funktion (i) hervorgehenden Kohle umfasst.

3. Vorrichtung (D) nach Anspruch 1 oder 2, **dadurch gekennzeichnet, dass** sie weiter eine Funktion SP (10) zum Trennen der aus der Funktion (iv) hervorgehenden Gase umfasst, um Wasserstoff zu gewinnen.

4. Vorrichtung (D) nach einem der vorstehenden Ansprüche, **dadurch gekennzeichnet, dass** sie weiter eine Funktion SP (11) zum Trennen der aus der Funktion (v) hervorgehenden Gase umfasst, um Methan zu gewinnen.

5. Vorrichtung (D) nach einem der vorstehenden Ansprüche, **dadurch gekennzeichnet, dass** die Biomethanisierungsfunktion B (5) über eine Biomethanisierungs-Untereinheit bewerkstelligt wird, die aus einem oder mehreren anaeroben Faulbehältern besteht.

6. Vorrichtung (D) nach einem der vorstehenden Ansprüche, **dadurch gekennzeichnet, dass** sie weiter einen Kreis (7) umfasst, der die Kondensate (8) stromaufwärts und/oder in die Pyrovergasungsfunktion Pygaz (1) leiten kann.

7. Vorrichtung (D) nach einem der vorstehenden Ansprüche, **dadurch gekennzeichnet, dass** sie weiter einen Kreis (7) umfasst, der die Kondensate (8) stromaufwärts und/oder in die Dampfreformierungsfunktion VR (2) leiten kann.

8. Vorrichtung (D) nach einem der vorstehenden Ansprüche, **dadurch gekennzeichnet, dass** sie weiter einen Kreis (7) umfasst, der die Kondensate (8) stromaufwärts und/oder in die Wassergas-Shift-Reaktionsfunktion WGS (4) leiten kann.

9. Vorrichtung (D) nach einem der vorstehenden Ansprüche, **dadurch gekennzeichnet, dass** die Kühl- und Waschfunktion RL (4) mittels einer Waschflüssigkeit, ausgewählt aus Wasser oder einem organischen Teerlösungsmittel, ausgeführt wird.

10. Verfahren zur Umwandlung von organischen Feststoffen in Gemische aus Methan (CH₄) und/oder Wasserstoff (H₂) und/oder Kohlendioxid (CO₂), das die folgenden aufeinanderfolgenden Schritte umfasst:
- einen Schritt des Beschickens einer Vorrichtung (D), wie nach einem der Ansprüche 1 bis 9 definiert, mit organischen Feststoffen,
- einen von der Funktion Pygaz (1) ausgeführten Schritt der Pyrovergasung der organischen Feststoffe, um ein Pyrovergasungsgas GP (6) zu bilden,
- einen von der Funktion VR (2) ausgeführten Schritt der Dampfreformierung der im Pyrovergasungsgas GP (6) enthaltenen Pyrovergasungsöle in Gegenwart von Wasser,
- einen von der Funktion WGS (3) ausgeführten Schritt der Wassergas-Shift-Reaktion der aus dem Schritt der Dampfreformierung in Gegenwart von Wasser hervorgehenden Gase,
- einen von der Funktion RL (4) ausgeführten Schritt des Kühlens und Waschens der aus dem Schritt der Wassergas-Shift-Reaktion hervorgehenden Gase,
- einen von der Biomethanisierungsfunktion B (5) ausgeführten Schritt der Biomethanisierung der aus dem Kühl- und Waschschritt hervorgehenden Gase.

11. Verfahren nach Anspruch 10, **dadurch gekennzeichnet, dass** es einen Schritt der Dampfvergasung der aus der Funktion Pygaz (1) hervorgehenden Kohle umfasst.

12. Verfahren nach Anspruch 10, **dadurch gekennzeichnet, dass** es stromabwärts des Kühl- und Waschschritts weiter einen Schritt umfasst, der darauf abzielt, den Wasserstoff und den Rest der Gase, darunter das Kohlendioxid, in den aus dem Kühl- und Waschschritt hervorgehenden Gasen zu trennen.

13. Verfahren nach Anspruch 10, **dadurch gekennzeichnet, dass** es weiter einen Trennschritt umfasst, der darauf abzielt, das Methan und den Rest der Gase, darunter das Kohlendioxid, in den aus dem Biomethanisierungsschritt hervorgehenden Biogasen zu trennen.

14. Verfahren nach einem der Ansprüche 10 bis 13, **dadurch gekennzeichnet, dass** es weiter einen Schritt des Gewinnens der aus dem Kühl- und Waschschritt hervorgehenden Kondensate (8) umfasst, um diese vor oder im Laufe des Pyrovergasungsschritts zu injizieren.

15. Verfahren nach einem der Ansprüche 10 bis 14, **dadurch gekennzeichnet, dass** es weiter einen Schritt des Gewinnens der aus dem Kühl- und Waschschritt hervorgehenden Kondensate (8) umfasst, um diese vor oder im Laufe des Dampfreformierungsschritts zu injizieren.

16. Verfahren nach einem der Ansprüche 10 bis 15, **dadurch gekennzeichnet, dass** es weiter einen Schritt des Gewinnens der aus dem Kühl- und Waschschritt hervorgehenden Kondensate (8) umfasst, um diese vor oder im Laufe des Wassergas-Shift-Reaktionsschritts zu injizieren.

17. Verfahren nach einem der Ansprüche 10 bis 16, **dadurch gekennzeichnet, dass** es weiter einen Schritt des Gewinnens der aus dem Kühl- und Waschschritt hervorgehenden Wärme umfasst, um diese vor oder im Laufe des Pyrovergasungsschritts zurückzuleiten.

18. Verfahren nach einem der Ansprüche 10 bis 17, **dadurch gekennzeichnet, dass** es weiter einen Schritt des Gewinnens der aus dem Kühl- und Waschschritt hervorgehenden Wärme umfasst, um diese vor oder im Laufe des Wassergas-Shift-Reaktionsschritts zurückzuleiten.

19. Verfahren nach einem der Ansprüche 10 bis 18, **dadurch gekennzeichnet, dass** es weiter einen Schritt des Gewinnens der aus dem Wassergasschritt hervorgehenden Wärme umfasst, um diese vor oder im Laufe des Pyrovergasungsschritts zurückzuleiten.

20. Verfahren nach einem der Ansprüche 10 bis 19, **dadurch gekennzeichnet, dass** der Kühl- und Waschschritt RL mittels einer oder mehrerer Waschflüssigkeiten ausgeführt wird, ausgewählt aus Wasser oder einem organischen Lösungsmittel der Teere, die in dem Pyrovergasungsgas GP (6) vorhanden sind, das aus dem von der Funktion Pygaz (1) ausgeführten Schritt der Pyrovergasung der organischen Feststoffe hervorgeht.

21. Verfahren nach einem der Ansprüche 10 bis 20, **dadurch gekennzeichnet, dass** der Kühl- und Waschschritt RL in zwei Schritten ausgeführt wird: einem Schritt des Kühlens und Waschens mit Wasser, und einem Schritt des Waschens mittels einer oder mehrerer Waschflüssigkeiten, ausgewählt aus den organischen Lösungsmitteln der Teere, die in dem Pyrovergasungsgas GP (6) vorhanden sind, das aus dem von der Funktion Pygaz (1) ausgeführten Schritt der Pyrovergasung der organischen Feststoffe hervorgeht.

## Claims

1. Device (D) for converting solid organic matter into mixtures of methane (CH₄) and/or hydrogen (H₂) and/or carbon dioxide (CO₂) comprising at least the five following functions:
(i) a pyrogasification function Pygaz (1) of solid organic matter in order to form a pyrogasification gas GP (6),
(ii) a steam reforming function VR (2) of the pyrogasification oils contained in the pyrogasification gas GP (6) resulting from function (i),
(iii) a gas-to-water reaction function WGS (3) applied to the gases resulting from function (ii),
(iv) a cooling and washing function RL (4) of the gases resulting from function (iii),
(v) a biomethanation function B (5) of the gases resulting from function (iv).

2. Device (D) according to claim 1, **characterised in that** it also comprises a steam gasification function of the chars resulting from function (i).

3. Device (D) according to claim 1 or 2, **characterised in that** it also comprises a separation function SP (10) of the gases resulting from function (iv) in order to recover hydrogen.

4. Device (D) according to any of the preceding claims, **characterised in that** it also comprises a separation function SP (11) of gases resulting from function (v) in order to recover methane.

5. Device (D) according to any of the preceding claims, **characterised in that** the biomethanation function B (5) is performed by a biomethanation subunit formed by one or more anaerobic digesters.

6. Device (D) according to any of the preceding claims, **characterised in that** it also comprises a circuit (7) able to channel the condensates (8) upstream and/or into the pyrogasification function Pygaz (1).

7. Device (D) according to any of the preceding claims, **characterised in that** it also comprises a circuit (7) capable of channelling condensates (8) upstream and/or into the steam reforming function VR (2).

8. Device (D) according to any of the preceding claims, **characterised in that** it also comprises a circuit (7) capable of channelling condensates (8) upstream and/or into the gas-to-water reaction function WGS (4).

9. Device (D) according to any of the preceding claims, **characterised in that** the cooling and washing function RL (4) is performed by means of a washing liquid selected from water or an organic tar solvent.

10. Method for converting solid organic matter into mixtures of methane (CH₄) and/or hydrogen (H₂) and/or carbon dioxide (CO₂) comprising the following successive steps:
- a step of supplying a device (D), as defined according to any of claims 1 to 9, with solid organic matter,
- a step of pyrogasification of the solid organic matter, performed by the Pygaz function (1), to form a pyrogasification gas GP (6),
- a steam reforming step, performed by the VR function (2), of pyrogasification oils contained in the pyrogasification gas GP (6) in the presence of water,
- a gas-to-water reaction step, performed by the WGS function (3), of gases resulting from the step of steam reforming in the presence of water,
- a cooling and washing step, performed by the RL function (4), of gases resulting from the gas-to-water reaction step,
- a biomethanation step, performed by the biomethanation function B (5), of gases resulting from the cooling and washing step.

11. Method according to claim 10, **characterised in that** it comprises a step of steam gasification of chars resulting from the Pygaz function (1).

12. Method according to claim 10, **characterised in that** it also comprises, downstream of the cooling and washing step, a step involving separating the hydrogen and remainder of gases, including carbon dioxide, among the gases resulting from the cooling and washing step.

13. Method according to claim 10, **characterised in that** it also comprises a separation step, involving separating the methane and the remainder of the gases, including carbon dioxide, among the biogases resulting from the biomethanation step.

14. Method according to any of claims 10 to 13, **characterised in that** it also comprises a step of recovering condensates (8) resulting from the cooling and washing step, to be injected before or during the pyrogasification step.

15. Method according to any of claims 10 to 14, **characterised in that** it also comprises a step of recovering condensates (8) resulting from the cooling and washing step, to be injected before or during the steam reforming step.

16. Method according to any of claims 10 to 15, **characterised in that** it also comprises a step of recovering condensates (8) resulting from the cooling and washing step, to be injected before or during the gas-to-water reaction step.

17. Method according to any of claims 10 to 16, **characterised in that** it also comprises a step of recovering the heat resulting from the cooling and washing step, to be returned before or during the pyrogasification step.

18. Method according to any of claims 10 to 17, **characterised in that** it also comprises a step of recovering the heat resulting from the cooling and washing step, to be returned before or during the gas-to-water reaction step.

19. Method according to any of claims 10 to 18, **characterised in that** it also comprises a step of recovering the heat resulting from the gas-to-water step, to be returned before or during the pyrogasification step.

20. Method according to any of claims 10 to 19, **characterised in that** the cooling and washing step RL is performed by means of one or more washing liquids selected from water or an organic tar solvent present in the pyrogasification gas GP (6) resulting from the pyrogasification step of solid organic matter, performed by the Pygaz function (1).

21. Method according to any of claims 10 to 20, **characterised in that** the cooling and washing step RL is performed in two steps: a step of cooling and washing in water and a step of washing with one or more washing liquids selected from the organic tar solvents present in the pyrogasification gas GP (6) resulting from the pyrogasification step of solid organic matter, performed by the Pygaz function (1).
